# EUROPEAN PATENT SPECIFICATION

(11) **EP 1 538 162 B1**
(45) Date of publication and mention of the grant of the patent: **11.04.2012**
(21) Application number: 04024019.4
(22) Date of filing: 24.02.2000
(51) Int. Cl.: C07K 14/47, C12N 15/12, A61K 38/17

(54) **Compositions for altering mucus secretion**
Verbindungen zur Veränderung der Schleimsekretion
Compositions pour modifier la sécrétion du mucus

(30) Priority: 24.02.1999 US 256154
(43) Date of publication of application: 08.06.2005
(62) Divisional of application: 00912034.6
(73) Proprietor: NORTH CAROLINA STATE UNIVERSITY, Raleigh, NC 27695-7003 (US)
(72) Inventor: Li, Yuehua, Pearland, TX 77584 (US); Martin, Kinda D., Apex NC 27502 (US); Adler, Kenneth B., Raleigh NC 27613 (US)
(74) Representative: Ward, Siobhan

(56) References cited:
- GRAFF J M ET AL: "Protein kinase C substrate and inhibitor characteristics of peptides derived from the myristoylated alanine-rich C kinase substrate (MARCKS) protein phosphorylation site domain." THE JOURNAL OF BIOLOGICAL CHEMISTRY. 5 AUG 1991, vol. 266, no. 22, 5 August 1991 (1991-08-05), pages 14390-14398, XP002324392 ISSN: 0021-9258
- BLACKSHEAR P J: "The MARCKS family of cellular protein kinase C substrates." THE JOURNAL OF BIOLOGICAL CHEMISTRY. 25 JAN 1993, vol. 268, no. 3, 25 January 1993 (1993-01-25), pages 1501-1504, XP002324393 ISSN: 0021-9258
- HARLAN D M ET AL: "THE HUMAN MYRISTOYLATED ALANINE-RICH C KINASE SUBSTRATE MARCKS GENE MACS ANALYSIS OF ITS GENE PRODUCT PROMOTER AND CHROMOSOMAL LOCALIZATION" JOURNAL OF BIOLOGICAL CHEMISTRY, vol. 266, no. 22, 1991, pages 14399-14405, XP002324394 ISSN: 0021-9258
- ABDULLAH L H ET AL: "Protein kinase C and Ca2+ activation of mucin secretion in airway goblet cells." THE AMERICAN JOURNAL OF PHYSIOLOGY. JUL 1997, vol. 273, no. 1 Pt 1, July 1997 (1997-07), pages L201-L210, XP008045512 ISSN: 0002-9513
- KIM K C ET AL: "Airway goblet cell mucin: its structure and regulation of secretion." THE EUROPEAN RESPIRATORY JOURNAL : OFFICIAL JOURNAL OF THE EUROPEAN SOCIETY FOR CLINICAL RESPIRATORY PHYSIOLOGY. NOV 1997, vol. 10, no. 11, November 1997 (1997-11), pages 2644-2649, XP002324396 ISSN: 0903-1936

## Description

### Field of the Invention

The present invention relates to methods and compositions that are useful in regulating mucus secretion, and that may be utilized in treating medical conditions where it is desirable to increase or decrease mucus secretion.

### Background of the Invention

Mucus is a biological liquid that is capable of forming gels. It is a mixture of components, including water and secretory products from a variety of cells. Expectorated human airway mucus contains approximately 95% water and 5% solids; the solids contents include 2-3% proteins and glycoproteins, 1% lipids, and 1% minerals. See Boat et al., Biochemistry of Mucus, In: Airway Secretion, Takashima and Shimura (eds.), Marcel Dekker, 1994. Mucins, also called mucous glycoproteins or epithelial glycoproteins, are glycoconjugates characterized by numerous oligosaccharide side chains linked to a peptide core by N- and O-linkages.

In the airways, mucins are released onto the airway surface from goblet cells on the surface epithelium, and from mucus cells of submucosal glands. The total amount of surface liquid (mucus) in the airways is the result of the rate of mucus secretion in conjunction with the rate of clearance of mucus (by epithelial reabsorption, evaporation, ciliary transport, and cough transport). Under "normal" conditions, the rate of secretion and clearance of mucus are balanced so that only a thin surface layer of liquid covers the tracheobronchial tree. Mucus hypersecretion (if not accompanied by a concomitant increase in mucus clearance) results in accumulation of airway mucus, which can result in airflow obstruction and increased retention of inhaled particulate matter and microbial matter. Existing strategies to reduce luminal mucus in the airways include inhibition of mucus hypersecretion using indirect pharmacological action, changing the physical characteristics of mucus to enhance ciliary action, and enhancement of cough clearance of mucus.

Hypersecretion of mucus contributes to the pathogenesis of a large number of airway inflammatory diseases in both human and non-human animals. Increased mucus secretion is seen in chronic disease states such as asthma, COPD and chronic bronchitis; in genetic diseases such as cystic fibrosis; in allergic conditions (atopy, allergic inflammation); in bronchiectasis; and in a number of acute, infectious respiratory illnesses such as pneumonia, rhinitis, influenza or the common cold. Accordingly, new methods and therapeutic compounds able to decrease or lessen mucus secretion are desirable.

Under-secretion of mucus also has harmful effects. Airway mucus acts as a physical barrier against biologically active inhaled particles, and may help prevent bacterial colonization of the airways and inactivate cytotoxic products released from leukocytes. King et al., Respir. Physiol. 62:47-59 (1985); Vishwanath and Ramphal, Infect. Immun. 45:197 (1984); Cross et al., Lancet 1:1328 (1984). In the eye, mucus maintains the tear film, and is important for eye health and comfort. Mucus secretion in the gastrointestinal tract also has a cytoprotective function. The role of mucus as a chemical, biological and mechanical barrier means that abnormally low mucus secretion by mucous membranes is undesirable.

In view of the foregoing, improved methods and compositions able to alter (*i.e.*, increase or decrease) mucus secretion from epithelial cells and mucus membranes are desirable.

### Summary of the Invention

The present invention provides a peptide consisting of from 5 to fewer than 50 contiguous amino acids from an N-terminal sequence of SEQ ID NO: 4, wherein said peptide Inhibits MARCKS protein-related mucus secretion and wherein said peptide reduces mucus secretion by a mucus-secreting cell as compared to that which would occur in the absence of said peptide.

An alternative embodiment of the invention provides a peptide consisting of from 10 to 50 contiguous amino acids from an N-terminal sequence of SEQ ID NO: 4, wherein said peptide inhibits MARCKS protein-related mucus secretion and wherein said peptide reduces mucus secretion by a mucus-secreting cell as compared to that which would occur in the absence of said peptide.

The peptide of the present invention blocks the secretion of inflammatory mediators from cells and decreases inflammation.

In one embodiment of the Invention, the peptide of the invention is myristoylated. In an alternative embodiment of the invention, the peptide has an amino acid sequence comprising SEQ ID NO:1.

In another embodiment the invention provides a myristoylated peptide of the N-terminal region of MARCKS protein wherein the peptide consists of an amino acid sequence that is identical to a contiguous sequence of 10 to 50 amino acids from an N-terminal sequence of SEQ ID NO: 4 and wherein said peptide reduces mucus secretion by a mucus-secreting cell as compared to that which would occur In the absence of said peptide.

In an alternative embodiment the invention provides a myristoylated peptide consisting of sequence; Myristic acid-GAQFSKTAAKGEAAAERPGEAAVA (SEQ ID NO:1) or myristoylated fragments thereof consisting of at least 10 amino acids. The peptide inhibits MARCKS protein-related mucus secretion and reduces mucus secretion by a mucus-secreting cell as compared to that which would occur in the absence of said peptide.

Another embodiment of the invention provides myristoylated peptide consisting of sequence; Myristic acid-GAQFSKTAAKGEAAAERPGEAAVA (SEQ ID NO:1) or myristoylated fragments thereof consisting of at least 5 amino acids. The peptide inhibits MARCKS protein-related mucus secretion and reduces mucus secretion by a mucus-secreting cell as compared to that which would occur in the absence of said peptide.

The invention also provides a peptide of the invention for use as a medicament and a pharmaceutical composition comprising the peptide of the invention together with a pharmaceutically acceptable carrier. In one embodiment the invention provides for the use of a peptide of the invention for the manufacture of a medicament for the therapeutic or prophylactic treatment of mucus secretion or inflammation. The invention also provides the use of a peptide for the manufacture of a medicament for the therapeutic or prophylactic treatment of mucus secretion and inflammation. The invention also provides the use of a peptide of the invention for the manufacture of a medicament for the therapeutic or prophylactic treatment of airway disease. A peptide of the invention is also provided for use in treating mucus secretion or inflammation. Alternatively a peptide of the invention is provided for use in treating mucus secretion and inflammation. A peptide of the invention is also provided for use in treating airway disease.

### Brief Description of the Drawings

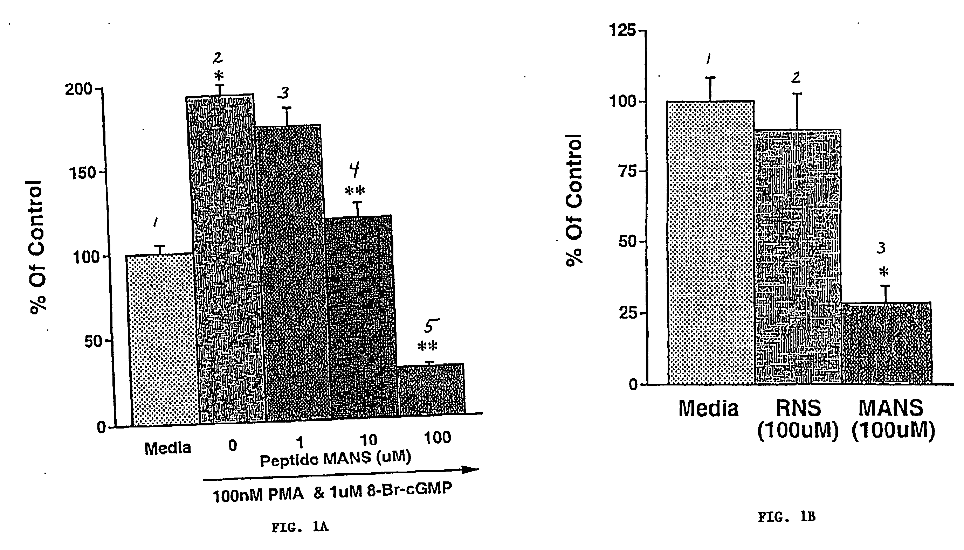

**Figure 1A** is a graph of stimulated mucin secretion by human bronchial epithelial cells *in vitro* in response to varying amounts of the MANS peptide (SEQ ID NO:1). Column 1 = media/control (no peptide, no stimulation); column 2 = 100 nM PMA and 1 µM 8-Br-cGMP (stimulated secretion); column 3 = 1 m µM MANS peptide, 100 nM PMA and 1 µM 8-Br-cGMP; column 4 = 10 µM MANS peptide, 100 nM PMA and 1 µM 8-Br-cGMP; and column 5 = 100 µM MANS peptide, 100 nM PMA and 1 µM 8-Br-cGMP. Single asterisks (*) indicate that the measured response was statistically different than the media control (column 1), and double asterisks (**) indicate that the response was statistically different than that of stimulated cells that were not exposed to the MANS peptide (column 2).
**Figure 1B** graphs the inhibition of basal mucin secretion by human bronchial epithelial cells exposed to the MANS peptide (SEQ ID NO:1) or a negative control peptide consisting of the same amino acids as the MANS peptide, but in random order (RNS peptide; random N-terminal sequence). Column 1 = media control; column 2 = one hour incubation with 100 µM of RNS; column 3 = one hour incubation with MANS peptide. The single asterisk (*) indicates that the response in column 3 was statistically different than the media control (column 1).

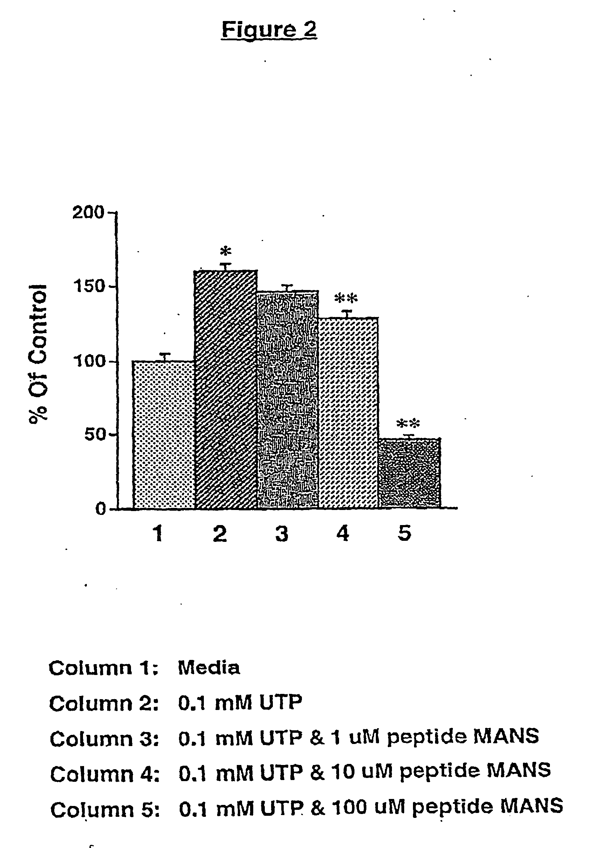

**Figure 2** is a graph of the effects of varying amounts of the MANS peptide (SEQ ID NO:1) on UTP-induced mucin secretion by human bronchial epithelial cells *in vitro.* Column 1 is the media/control; column 2 = 0.1 mM UTP; column 3 = 0.1 mM UTP and 1 µM MANS peptide; column 4 = 0.1 mM UTP and 10 µM MANS peptide; and column 5 = 0.1 mM UTP and 100 µM MANS peptide. Single asterisks (*) indicate that the measured response was statistically different than the media control (column 1), and double asterisks (**) indicate that the response was statistically different than that of UTP-stimulated cells that were not exposed to the MANS peptide (column 2).

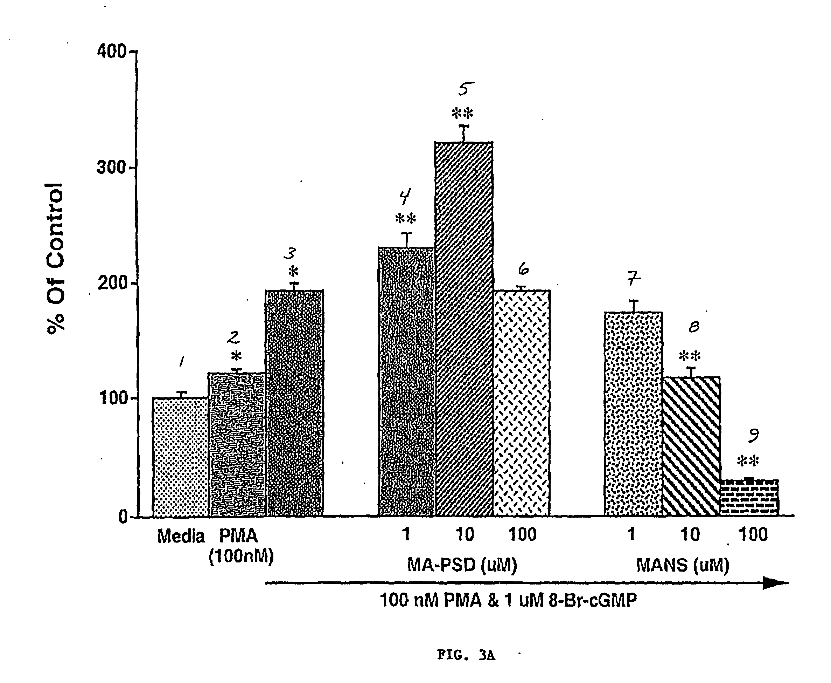

**Figure 3A** is a graph of the effects of the MANS peptide (SEQ ID NO:1) and the MA-PSD peptide (SEQ ID NO:2) on stimulated mucin secretion by human bronchial epithelial cells *in vitro.* Column 1 = media/control; column 2 = 100 nM PMA; column 3 = 100 nM PMA and 1 µM 8-Br-cGMP; column 4 = 100 nM PMA, 1 µM 8-Br-cGMP and 1 µM MA-PSD peptide; column 5 = 100 nM PMA, 1 µM 8-Br-cGMP and 10 µM MA-PSD peptide; column 6 = 100 nM PMA, 1 µM 8-Br-cGMP and 100 µM MA-PSD peptide; column 7 = 100 nM PMA, 1 µM 8-Br-cGMP and 1 µM MANS peptide; column 8 = 100 nM PMA, 1 µM 8-Br-cGMP and 10 µM MANS peptide; and column 9 = 100 nM PMA, 1 µM 8-Br-cGMP and 100 µM MANS peptide. Single asterisks (*) indicate that the response was statistically different than the media control (column 1), and double asterisks (**) indicate that the response was statistically different than that in stimulated cells that were not exposed to the MANS peptide (column 3).

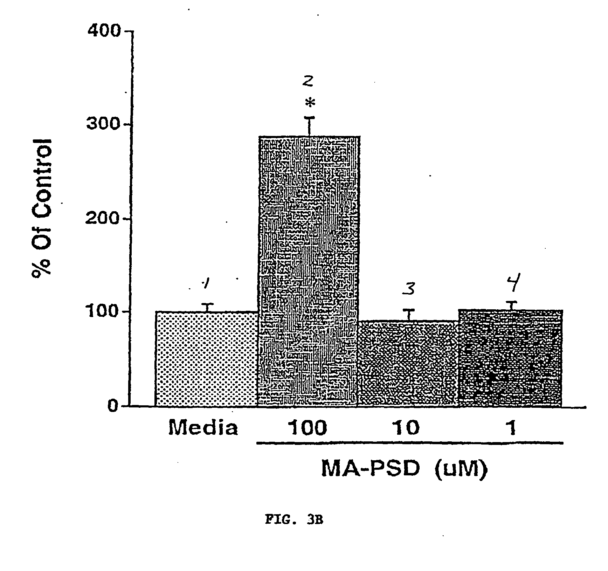

**Figure 3B** graphs the effect of one hour of incubation with the MA-PSD peptide (SEQ ID NO:2) on basal mucin secretion by human bronchial epithelial cells *in vitro.* Column 1 = media/control; column 2 = 100 µM MA-PSD; column 3 = 10 µM MA-PSD; column 4 = 1 µM MA-PSD. The single asterisk (*) indicates that the response in column 3 was statistically different than the media control (column 1).

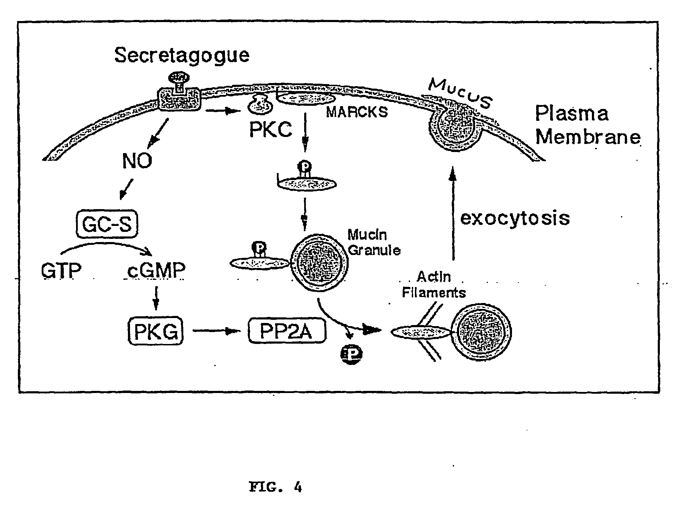

**Figure 4** is an illustration of a proposed signaling pathway of MARCKS-mediated mucin secretion by human epithelial cells. In this Figure, PKC = protein kinase C; PKG = cGMP-dependent protein kinase; GC-S = soluble guanylyl cyclase; PP2A = protein phosphatase 2A; NO = nitric oxide; GTP = guanosine triphosphate; and cGMP = cyclic guanosine monophosphate. In this proposed pathway, mucin secretagogues (shown in the Figure as binding to a receptor) interact with airway epithelial (goblet) cells and activate two separate protein kinases: PKC and PKG. Activated PKC phosphorylates MARCKS, causing its translocation from the plasma membrane to the cytoplasm, where it is targeted to the mucin granule membrane with the assistance of MARCKS-associated proteins. PKG, activated via the nitric oxide (NO)-cGMP-PKG pathway, in turn activates a cytoplasmic protein phosphatase 2A (PP2A), which dephosphorylates MARCKS, thus stabilizing its attachment to the granule membrane and allowing MARCKS to cross-link actin filaments. This tethers the granule to the cytoskeleton for movement and exocytosis.

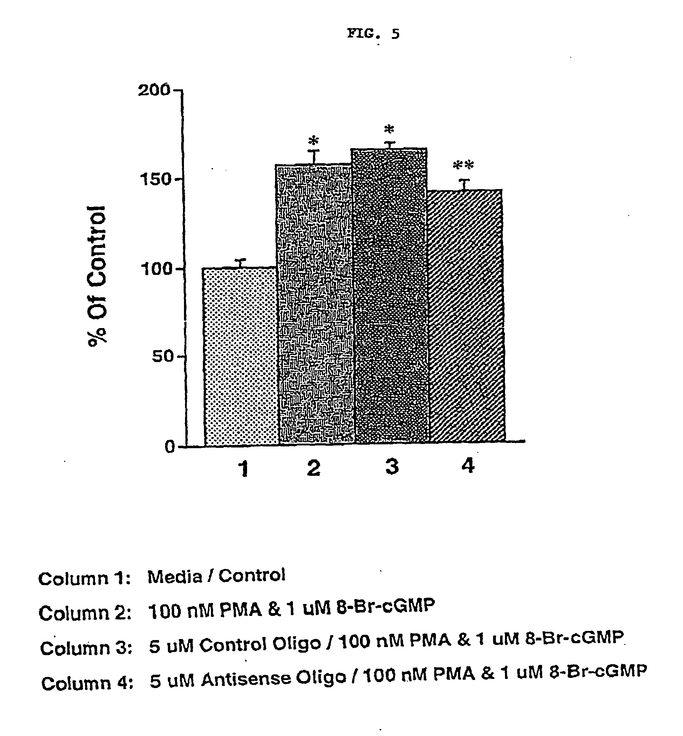

**Figure 5** is a graph of the effects of a MARCKS antisense oligonucleotide on stimulated mucin secretion by human bronchial epithelial cells *in vitro.* Column 1 = media/control; column 2 = cells stimulated with 100 nM PMA and 1 µM 8-Br-cGMP; column 3 = 5 µM control oligonucleotide, 100 nM PMA and 1 µM 8-Br-cGMP; column 4 = 5µM antisense oligonucleotide, 100 nM PMA and 1 µM 8-Br-cGMP. Single asterisks (*) indicate that the measured response was statistically different than the media control (column 1), and double asterisks (**) indicate that the response was statistically different than that observed in stimulated cells that were not exposed to the any oligonucleotide (column 2).

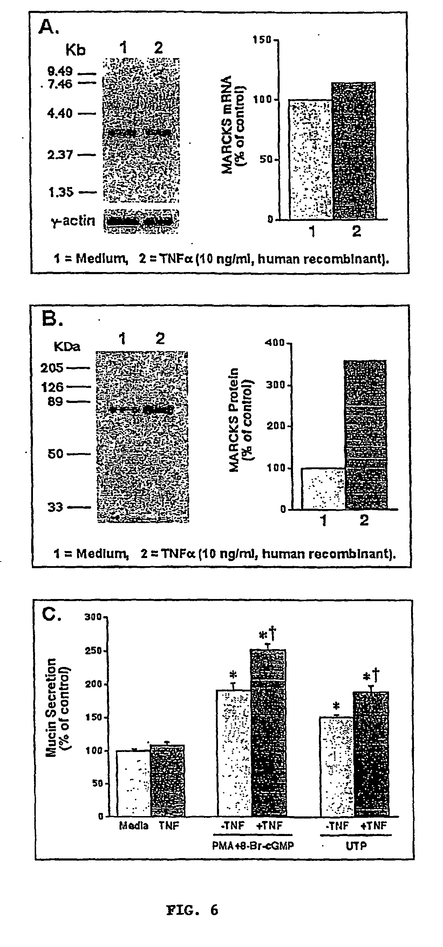

**Figures 6A, 6B**, **and 6C** together illustrate that TNF-α up-regulates MARCKS expression and augments mucin hypersecretion. NHBE cells were incubated with 10 ng/ml human recombinant TNF-α or medium alone for 4 hrs, then stimulated with PMA (100nM) + 8-Br-cGMP (1µM) for 15 min, or UTP (0.1 mM) for 2 hrs. Secreted mucin was collected and measured by ELISA. Total RNA and protein were isolated from treated cells. MARCKS mRNA was assessed by Northern hybridization, and MARCKS protein by the Western Blot technique.
**Figure 6A** is a Northern-blot and graph indicating an increase in MARCKS mRNA in cells incubated with TNF-α (lane 2 of blot, column 2 of graph) compared to cells incubated in medium alone (lane 1 of blot, column 1 of graph).
**Figure 6B** is a Westem-blot and graph showing a three- to four-fold increase in MARCKS protein in cells incubated with TNF-α (lane 2 of blot, column 2 of graph) as compared with cells incubated with medium only (lane 1 of blot, column 1 of graph).
**Figure 6C** is a graph showing that in cells incubated with TNF-α, mucin hypersecretion was significantly augmented in response to subsequent stimulation by PMA + 8-Br-cGMP or UTP when compared to mucin secretion of cells incubated in medium only. Data are presented as mean ± SEM (n=6 at each point). Single asterisks (*) indicate a statistically significant difference from control (medium-only) samples (*p*<0.05). Single cross marks (†) indicate a statistically significant difference from stimulus (*p*<0.05).

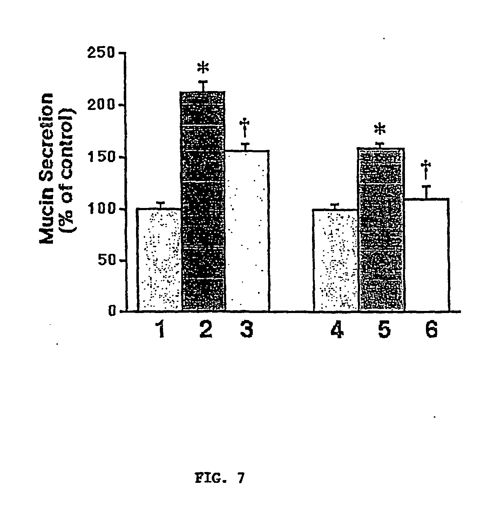

**Figure 7** is a graph showing that okadaic acid, a phosphatase inhibitor, blocks mucin hypersecretion induced by PMA + 8-Br-cGMP or UTP. NHBE cells were pre-incubated with okadaic acid (500nM) for 15 min at 37°C/5%CO₂, then stimulated with PMA (100 nM) + 8-Br-cGMP (1µM) for 15 min, or with UTP (0.1 mM) for 2 hours. Secreted mucin in the apical medium was collected and assayed by ELISA. Column 1 shows the results of incubation with medium alone for 30 min. Column 2 shows the results of pre-incubation with medium alone for 15 min, then incubation with PMA + 8-Br-cGMP for an additional 15 min. Column 3 shows the results of pre-incubation with okadaic acid for 15 min, then co-incubation with PMA + 8-Br-cGMP for an additional 15 min. Column 4 shows the results of incubation with medium alone for 2 hrs. Column 5 shows the results of pre-incubation with medium alone for 15 min, then incubation with UTP for an additional 2 hrs. Column 6 shows the results of pre-incubation with okadaic acid for 15 min, then co-incubation with UTP for an additional 2 hrs. Data are presented as mean ± SEM (n=6 at each point). Single asterisks (*) indicate a statistically significant difference from control (*p*<0.05). Single cross marks (†) indicate statistically significant difference from stimulus (*p*<0.05).

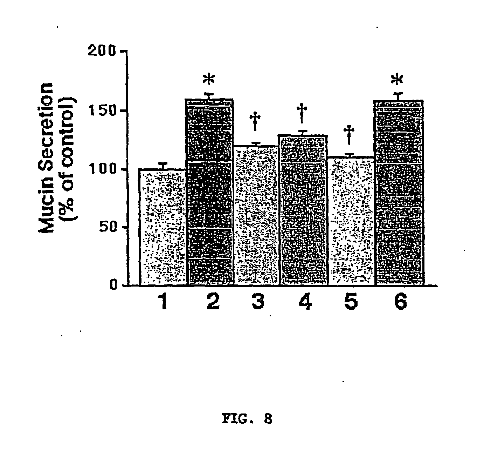

**Figure 8** is a graph showing that mucin hypersecretion induced by UTP involves activation of PKC and PKG. NHBE cells were pre-incubated with the indicated inhibitor (described below) for 15 min, then stimulated with UTP (0.1mM) for 2 hours. Secreted mucin in the apical medium was collected and assayed by ELISA. Column 1 indicates the results of incubation with medium alone. Column 2 indicates the results of incubation with 0.1 mM UTP. Column 3 indicates the results of incubation with 0.1mM UTP + 500 nM calphostin C (an inhibitor of PKC). Column 4 indicates the results of incubation with 0.1 mM UTP + 10 µM Rp-8-Br-PET-cGMP (an inhibitor of PKG). Column 5 indicates the results of incubation with 0.1 mM UTP + 50 µM LY83583 (an inhibitor of soluble guanylyl cyclase). Column 6 indicates the results of incubation with 0.1 mM UTP + 500 nM KT5720 (an inhibitor of PKA). Data are presented as mean ± SEM (n=6 at each point). Single asterisks (*) indicate a statistically significant difference from control (*p*<0.05). Single cross marks (†) indicate a statistically significant difference from UTP stimulation (*p*<0.05).

### Detailed Description of Preferred Embodiments

Mucus is the clear viscous secretion of the mucous membranes, and comprises water, mucin, lipids, and various inorganic salts. Mucin is a carbohydrate-rich glycoprotein that is secreted by specialized epithelial cells (known as goblet cells), the submaxillary glands, and other mucous glandular cells. Goblet cells are epithelial cells specialized for secretion and containing an accumulation of mucous secretory granules.

Mucous tissue (or mucosa) lines various anatomic structures in the mammalian and avian body, including the eyes, respiratory tract (alveoli, bronchi, oral cavity, larynx, nasal cavity, pharynx, trachea), gastrointestinal tract (esophagus, stomach, small and large intestine, rectum), and genitourinary tract (urethra, urinary bladder, uterus and vagina).

Alterations in the quantity of mucus secretions may be due to various underlying factors, including a change in the amount of mucous glycoproteins secreted from mucus-secreting cells, a change in the total number of mucus-secreting cells, or combinations thereof. Mediators released by the inflammatory response are known to act as mucus secretagogues, including lipid mediators, oxygen metabolites, and other cell-specific products. Larivee et al., In: Airway Secretion, Takishima and Shimura (Eds.), Marcel Dekker Inc., 1994, pages 469-511.

As used herein, "hypersecretion" of mucus refers to production of mucus above a normal or basal amount, or production of mucus in an amount that leads to pathological changes or symptoms.

As used herein, the "inhibiting mucus secretion" refers to a lessening or reduction in mucus secretion; it is not meant to imply the complete cessation of mucus secretion. A treatment that inhibits mucus secretion results in decreased mucus production compared to that which would occur, or would be expected, in the absence of such treatment.

Amounts of mucus secreted by a cell in culture, or by a tissue *in vivo* can be measured or assessed using methods as are known in the art.

As used herein, the "enhancement" or "stimulation" of mucus secretion refers to an increase in mucus secretion. A treatment that enhances mucus secretion results in increased mucus production compared to that which would occur, or would be expected, in the absence of such treatment.

As used herein, "stimulated mucus secretion" refers to mucus secretion that occurs in response to a secretagogue; this is contrasted to "basal mucus secretion" that occurs under normal physiological conditions.

As used herein, a compound that inhibits the MARCKS protein-mediated release of mucin granules (or mucus) includes those compounds that act upon a step in the MARCKS protein-mediated signaling pathway that results in mucus secretion, causing a reduction in mucus secretion.

As used herein, "endogenous" refers to compounds that are naturally occurring in a cell. Endogenous MARCKS protein thus refers to MARCKS protein that is found within a cell, as opposed to MARCKS protein introduced into that cell (either administered directly or by genetic engineering techniques).

As used herein, an "active fragment" of a MARCKS protein is one that affects (inhibits or enhances) the MARCKS protein-mediated release of mucus that occurs in response to a secretagogue such as UTP (uridine 5'-triphosphate). An active peptide fragment of MARCKS comprises an amino acid sequence that is identical or substantially identical to a contiguous sequence of amino acids found in a naturally occurring MARCKS protein. Active MARCKS protein fragments are typically at least about five, ten, fifteen, twenty or twenty-five amino acids in length, but are shorter than the complete MARCKS protein. Active MARCKS protein fragments preferably have fewer than about fifty, seventy-five, one hundred or two hundred amino acids.

A "mucus inhibitory" or "mucus inhibiting" amount of a compound is that amount which reduces or inhibits mucus secretion, compared to that which would occur in the absence of the compound. A "mucus enhancing" amount of compound is that amount which enhances or increases mucus secretion, compared to that which would occur in the absence of the compound. For example, as described herein, peptides of SEQ ID NO:2 were found to increase mucus secretion in airway epithelium *in vivo* when provided in a certain amount, and to inhibit mucus secretion when provided in greater amounts. The most effective amount of a particular peptide will vary depending upon the peptide, route of administration, and condition being treated. As used herein, the term "compound" is to be broadly construed to include proteins, peptide fragments, nucleotides, oligonucleotides, and other non-protein chemicals.

As used herein, a peptide inhibitor of MARCKS-related mucus secretion (or release of mucin) is a peptide that, when provided to a mucus secreting cell, inhibits or reduces the secretion of mucus compared to that which would occur in the absence of said peptide.

As used herein, a peptide enhancer of MARCKS-related mucus secretion (or mucin release) is a peptide that, when provided to a mucus secreting cell, enhances or increases the secretion of mucus compared to that which would occur in the absence of said peptide.

As used herein, "oligonucleotide" refers to DNA or RNA and can include sense and/or antisense strands as appropriate to the desired effect. Oligonucleotides useful in the present invention may be incorporated into recombinant expression vectors that include a promoter and other sequences necessary for expression of the desired translation products (such as a peptide). Alternatively, 'naked' oligonucleotides may be delivered to target cells, as is known in the art (see, e.g., Felgner et al., US Patent No. 5,580,859).

Mucosa or mucous membranes, as used herein, refers to mucosal tissues of a host wherever they may be located in the body including but not limited to respiratory passages (nasal, oral, tracheal, bronchial), genital passages (vaginal, cervical, anal and penile), urinary passages (urethra, bladder), and the eyes.

The present invention provides methods and compositions that are useful in inhibiting mucus secretion from epithelial cells. The present inventors have determined that mucin secretory processes in epithelial cells involve the protein kinase C (PKC) substrate MARCKS protein (myristolated alanine-rich C-kinase substrate). Groff et al., The Journal of Biological Chemistry vol.266, no.22:14390-14398 (1991) discloses that the phosphorylation sites in the MARCKS protein consist of four serines contained within a conserved, basic region of 25 amino acids, termed the phosphorylation site domain. By blocking or inhibiting the function and/or production of MARCKS protein in secretory epithelial cells, mucin secretion is reduced over that which would otherwise occur (i.e., that would occur in the absence of such treatment). Also described are methods and compositions that are useful in enhancing mucus secretion from epithelial cells. By enhancing the function and/or production of MARCKS protein in secretory epithelial cells, mucin secretion is increased over that which would otherwise occur (i.e., that would occur in the absence of said blocking).

The present inventors have shown that use of a fragment of the MARCKS protein reduces mucus secretion by epithelial cells. Additionally, use of antisense fragments directed against the MARCKS mRNA sequence also has been shown to decrease mucus production in epithelial cells.

Despite the previous identification of numerous mucus secretagogues, common signaling pathways and intracellular molecules involved in mucin secretion have not previously been elucidated. The present invention exploits the unexpected discovery that the myristolated alanine-rich C-kinase substrate (MARCKS) protein is involved in the secretory process of cells, and particularly in the secretion of mucus from epithelial cells (such as goblet cells). MARCKS protein is a major cellular substrate for protein kinase C (PKC), and the present inventors' studies indicate that it is a central, convergent molecule controlling release of mucin granules. While not wishing to be held to any single theory of the present invention, the MARCKS-related secretion of mucus appears to involve the interaction of mucin secretagogues with airway epithelial (goblet) cells and the activation of two separate protein kinases: PKC and PKG. Activated PKC phosphorylates MARCKS, causing its translocation from the plasma membrane to the cytoplasm, where it is targeted to the mucin granule membrane with the assistance of MARCKS-associated proteins. PKG, activated via the nitric oxide (NO)-cGMP-PKG pathway, in turn activates a cytoplasmic protein phosphatase 2A (PP2A), which dephosphorylates MARCKS, stabilizing its attachment to the granule membrane and allowing MARCKS to cross-link actin filaments, thereby tethering the granule to the cytoskeleton for movement and exocytosis. This proposed signaling pathway is generally depicted in **Figure 4**.

The present inventors identified MARCKS mRNA and protein in human bronchial epithelial cells, and both mRNA and protein levels increased with secretory cell differentiation *in vitro*. The MARCKS in these cells was phosphorylated by the phorbol ester PMA (phorbol 12-myristate 13-acetate), while subsequent addition of a cGMP activator (8-bromo-cGMP), caused dephosphorylation. Mucin secretion provoked (*i*.*e*., stimulated) by the pathophysiologically relevant secretagogue uridine triphosphate (UTP) (or by a combination of PMA and 8-bromo-cGMP) was inhibited in a dose-dependent manner by a myristoylated peptide fragment of the N-terminal region of MARCKS protein (the proposed site of the protein's attachment to granule membranes). Accordingly, this myristoylated peptide fragment of the N-terminal region of MARCKS protein, as well as other active peptide fragments, are useful in methods of inhibiting mucus secretion. As described further herein, the administration of certain active fragments of MARCKS protein has been found to be capable of both increasing and decreasing mucus secretion by epithelial mucus-secreting cells.

The present inventors have discovered that antisense oligonucleotides directed against MARCKS protein block or inhibit mucin secretion, as described further herein. Accordingly, such antisense oligonucleotides find use in methods of inhibiting mucus secretion.

Additionally, certain non-protein inhibitors of components in the mucus secretion signaling pathway illustrated in **Figure 4** inhibit mucus secretion in mucus-secreting cells, and are thus useful in the practice of methods the described herein. For example, inhibitors of PKC such as calphostin C, inhibitors of cyclic GMP such as Rp-8-Br-PET-cGMP, inhibitors of PKG such as Rp-8-Br-PET-cGMP, inhibitors of soluble guanylyl cyclase such as LY83585 and inhibitors of phosphatase such as okadaic acid each inhibit mucin secretion in cells stimulated by the above-listed secretagogues. Accordingly, such inhibitors of components of the mucin secretion signaling pathway find use in methods of inhibiting mucus secretion.

Other compounds that find use are those compounds that increase the amount (*i*.*e*., the concentration) of MARCKS protein in a mucus secreting cell. Such compounds have been found to increase mucus secretion in these cells. Although the mechanisms of how these compounds increase the level or amount of MARCKS protein in the cell are not known, possible mechanisms include (1) transcriptional modulation of the production of MARCKS mRNA (*i.e*., through binding or alteration of transcription factors, or other mechanisms of modulating transcription) by these compounds, directly or indirectly, and (2) translational control or modulation of the expression of MARCKS protein (*e.g*., by binding in the upstream regulatory region of the MARCKS mRNA, thus potentially altering secondary structure that inhibits the amount of MARCKS protein expressed) by these compounds, either directly or indirectly. As used herein, compounds that increase the amount of MARCKS protein in a mucus secreting cells may be proteins, peptides, or non-protein compounds. In one embodiment, the compound is bacterial lipopolysaccharide (LPS). Preferably, the compound is a protein or peptide. In a particularly preferred embodiment, the compound is a cytokine. In a more particularly preferred embodiment, the compound that increases the amount of MARCKS protein in a mucus secreting cell is the cytokine Tumor Necrosis Factor-alpha (TNF-α).

The present invention thus describes methods and compositions useful in regulating (increasing or decreasing) mucus secretion. Such methods and compositions are useful in the treatment of medical conditions in which mucus hypersecretion occurs, and are particularly useful in the respiratory tract. Methods and compositions may further be useful in treating medical conditions in which it is desired to increase mucus secretion.

The methods and compositions of the present invention may be used to inhibit or reduce mucus secretion occurring from any mucus-secreting cell (such as goblet cells) or tissue (such as mucous membranes of the airways). While not wishing to be held to a particular theory, the present inventors also believe that the compounds and methods may also be used to block the secretion of inflammatory mediators from cells such as macrophages, neutrophils and mast cells. In this way, the present mucus-inhibitory compounds may have a dual function of decreasing mucus secretion and inflammation.

As discussed below, also described are active peptide fragments of MARCKS protein that exhibit a bimodal effect when delivered to mucus-secreting cells. At a certain dose level (a mucus-enhancing amount), such peptides increase or enhance mucus secretion (compared to that which would occur in the absence of such treatment). At a different dose level, this enhancement is no longer observed. An even more extreme dose results in the inhibition of or decrease in mucus secretion (compared to that which would occur in the absence of such treatment).

Accordingly, described are methods and compositions for regulating mucus secretion, by regulating the effects of MARCKS protein in the mucus-secretory pathway. Such regulation can be achieved by administering active fragments of MARCKS protein in pre-determined amounts, administration of mucus-inhibiting amounts of MARCKS antisense oligonucleotides, or administration of these or other compounds (alone or in combination) that enhance or inhibit the MARCKS-related secretory pathway. Such compounds include those that block the dephosphorylated MARCKS protein binding event that leads to mucin release. Such compounds may bind to and block the site that is bound by endogenous MARCKS protein, or may bind to the MARCKS protein at the pertinent site. The MANS peptide described herein is believed to compete with endogenous MARCKS protein for the pertinent binding site in the cell, thus blocking the MARCKS-mediated release of mucin within the cell. Alternatively, an antibody directed to the N-terminal sequence of the MARCKS protein (e.g., the MANS sequence) would be predicted to bind to endogenous MARCKS protein and block binding.

While not wishing to be held to a single theory underlying the present invention, it is believed that compounds that increase MARCKS-related mucus secretion when administered to a mucus-secreting cell (such as the MA-PSD peptide; SEQ ID NO:2) may be binding to endogenous proteins in the cell that would otherwise bind to MARCKS protein and inhibit MARCKS from completing a step in the mucus-secretion pathway. Calmodulin is one such endogenous inhibitor of MARCKS; calmodulin binds to MARCKS and prevents phosphorylation, thus preventing the MARCKS protein from disengaging from the plasma membrane. As used herein, "endogenous inhibitors of MARCKS protein" are compounds naturally present in a cell that bind to MARCKS protein and prevent the completion of a step in the MARCKS-related mucus secretion pathway. A peptide or other compound that binds to a MARCKS inhibitor would leave more endogenous MARCKS protein free to function in the mucus secretion pathway. Thus, a method for increasing mucus secretion is to administer to a mucus-secreting cell, a compound that binds to a MARCKS protein inhibitor.

It will be desirable, in many therapeutic situations, to maintain some level of mucus secretion (i.e., a basal or normal level), for the protective effects of mucus. Maintenance of basal mucus secretion may be achieved by regulating the dose of the active compound utilized. Additionally, while not wishing to be held to a single theory of the invention, the present inventors suggest that in some mucous membranes, a basal level of mucus secretion may be maintained by a pathway separate from the MARCKS-related pathway and stimulated mucus secretion.

The present invention provides methods and compositions able to decrease or reduce mucus hypersecretion that occurs in many pathological conditions, including pathological conditions related to inflammatory, viral, bacterial, or genetic causes. In particular, the present methods and compositions provide methods of treating airway diseases in which mucus secretion is increased over that which occurs in the absence of the disease (i.e., is increased over basal levels, or over normally-occurring levels of mucus secretion). Subjects to be treated by the present methods include human and non-human subjects. Non-human subjects include companion animals such as cats and dogs, as well as livestock such as cattle, horses, sheep and swine.

The present methods and compositions may be used to reduce mucus secretion, or to inhibit mucus hypersecretion, in any secretory epithelium, or epithelial cell, including but not limited to airway epithelial cells (e.g., oral, nasal, bronchial), ocular epithelial cells, gastric or intestinal epithelial cells, and epithelial cells lining the reproductive tract (e.g., vaginal, cervical). As will be apparent to those skilled in the art based on the subject and the condition being treated, it may be desirable to maintain a basal level of mucus secretion, while reducing hypersecretion of mucus. As used herein, a treatment that reduces or inhibits mucus secretion refers to a treatment that reduces the amount of secreted mucus compared to that which would occur in the subject in the absence of such treatment.

Further described is a method and compositions for increasing or stimulating mucus secretion by epithelial cells, including but not limited to airway epithelial cells, ocular epithelial cells (corneal epithelium or conjunctiva), gastric or intestinal epithelial cells, and epithelial cells lining the reproductive tract. As used herein, a treatment that increases, enhances or stimulates mucus secretion refers to a treatment that increases the amount of secreted mucus compared to that which would occur in the absence of such treatment. In particular, the present methods and compositions are useful in increasing the secretion of mucus by ocular epithelial cells (to treat dry-eye conditions), and by vaginal epithelial cells (to treat vaginal dryness).

The peptides and compounds of the present invention block mucus secretion in response to known activators of PKC and protein kinase G (**Figure 1**), and to physiologically relevant stimuli (e.g., UTP). (**Figure 2**)

The present invention thus provides methods and compositions for treating epithelial cells or epithelial tissue, where it is desirable to decrease the amount of mucus secreted by the cells or tissue. Also described are methods and compositions for treating epithelial cells or epithelial tissue, where it is desirable to increase the amount of mucus secreted by the cells or tissue. In particular the present invention provides methods and compositions for treating respiratory conditions where it is desirable to decrease the amount of mucus present in the airways, or where it is desirable to increase the amount of mucus present in the airways. Conditions suitable for treatment by the present methods include human and animal inflammatory, viral or bacterial airway disease (e.g., asthma, chronic obstructive pulmonary disease, common cold, rhinitis, acute or chronic bronchitis, pneumonia, and kennel cough), allergic conditions (atopy, allergic inflammation), bronchiectasis, and certain genetic conditions (e.g., cystic fibrosis).

### Mucus Secretion in the Airways

Normal mucus secretion in the lung plays an important role in clearing inhaled foreign particles and pathogens from the airways. Mucus traps inhaled particles, and is then removed from the airways by ciliary action or by coughing. Above-normal levels of mucus secretion (hypersecretion) in the airways can lead to intraluminal mucus accumulation, resulting in airflow obstruction and an increased susceptibility to infectious agents. Secretory cells in the airways include submucosal glands and superficial epithelial mucus cells (goblet cells).

Airway mucus secretion is an important determinant in the prognosis and clinical features of pulmonary diseases. Hypertrophy and/or hyperplasia of airway secretory cells (bronchial glands and epithelial goblet cells) are often found in conditions associated with chronic airway inflammation. In subjects with chronic bronchitis and bronchial asthma, goblet cell hyperplasia has been observed, with a two- to three-fold increase in the numbers of goblet cells compared to controls. Cutz et al., Histopathology 2:407-421 (1978), Glynn & Michaels Thorax 15:142-153 (1960). Inflammation of the airways may induce mucus hypersecretion by multiple mechanisms, including the release of chemical mediators from surrounding tissues and cells. Airway mucus hypersecretion is a particularly dominant clinical finding in cystic fibrosis, bronchitis, chronic obstructive pulmonary disease (COPD), emphysema, and bronchial asthma. *See, e.g.,* Airway Secretion, Takishima and Shimura (Eds.), Marcel Dekker Inc., 1994. The presence of excessive bronchial mucus can lead to respiratory failure and bacterial infection. Lungs of asthmatic patients, at autopsy, often show the presence of excessive bronchial mucus and mucus plugging. Methods of reducing airway mucus secretion would be useful for the treatment of such conditions, as well as in treating bacterial or viral infections (e.g., pneumonia, influenza, and the common cold); in animals, such methods are further useful in treating kennel cough and equine COPD.

Various methods are currently in use to reduce mucus secretion when needed in disease states. Some therapies act to decrease the signals or stimuli that upregulate mucus secretion. For example, inflammatory mediators may upregulate mucus secretion; steroid treatments are often used to decrease inflammation and thus indirectly decrease mucus secretion. Antihistamines are used to block the responses to allergens which can trigger attacks of allergic asthma. The thickened mucus present in patients with cystic fibrosis is removed by compression therapy, and infections occurring due to the thickened mucus are treated with antibiotics. The methods and compounds of the present invention vary from the above treatments in that cellular secretion of mucus in response to a variety of stimuli is directly blocked at the cellular level.

### Ocular Mucus Layer

The mucus layer on the ocular surface is important in maintaining and spreading the tear film, and is required for normal functioning of the eyes. The ocular surface epithelia has been shown to express multiple mucin genes. Gipson, Adv. Exp. Med Biol. 438:221 (1998). Various diseases and syndromes result in pathological "dry-eye" conditions, including keratoconjunctivitis sicca, Stevens-Johnson syndrome, ocular pemphigoid, and surgery- or radiation-induced dry-eye. The ocular surface epithelia in such diseases undergoes changes, which may include loss of goblet cells, mucin deficiency, and keratinization. Lower goblet cell densities in the ocular epithelia have been demonstrated in these syndromes. Ralph, Invest. Ophthalmol. 14:299 (1975).

In addition to subjects in which dry-eye causes discomfort in daily life, many individuals have "marginal" dry-eye which may only present difficulties when the subject attempts to wear contact lenses. Contact lens intolerance is frequently due to insufficient tear film. Jurkus et al., J. Am. Optom. Assoc. 65:756 (1994); Toda et al., Br. J. Ophthal. 80:604 (1996). Existing treatments for dry-eye include topical use of tretinoin (Tseng, J. Am. Acad. Dermatol. 15:860 (1986)) or retinoic acid (Driot & Bonne, Invest. Ophthalmol. 33:190 (1992)).

Increasing mucus secretion in the eyes is desirable where a lack of ocular mucus affects the normal function of the eye. Additionally, increasing mucus secretion in the eye is desirable as an aid in wearing contact lenses.

### Administration

The method described herein can be used to reduce (i.e., decrease or inhibit) or to enhance (i.e., increase or stimulate) the production of mucus secretions by mucous membranes or mucus-secreting cells, in a subject in need of such treatment for any reason. Using methods of administration as are known in the art, the present therapies can be directed to the mucous membranes or mucus-secreting cells of a particular target organ (including but not limited to the oral cavity, nasal cavity, lungs, gastrointestinal tract, eye and reproductive tract), in order to reduce or increase the amount of mucus secreted by, or retained upon, the surfaces being treated. The change (reduction or increase) in mucus is assessed by comparison to that which was present prior to treatment (or in the absence of treatment), or to that which would be expected in the absence of such treatment in view of the subject's condition.

The methods described herein may be used in conjunction with other therapies or compounds, including steps to remove retained mucus secretions from the airways of subjects prior to the step of administering the present compounds. This facilitates application of the active agent to the respiratory epithelia during the administering step. Such removal of retained mucus secretions can be carried out by any suitable physical or medicinal means as are known in the art.

Mucosal delivery of peptide-based drugs is discussed in Chien, Novel Drug Delivery Systems, Chapter 4 (Marcel Dekker, 1992); nasal drug delivery is discussed in Chien, supra, in Chapter 5. See also Chang et al., Nasal Drug Delivery, "Treatise on controlled Drug Delivery", Chapter 9 (Marcel Dekker, 1992). Agents known to enhance absorption of drugs through the skin are described in Sloan, Chapter 5, "Prodrugs: Topical and Ocular Drug Delivery" (Marcel Dekker, 1992). The peptides of the present invention can be administered into target cells directly, for example using liposomes. It is expected that those skilled in the art may adapt such techniques and other known drug delivery techniques for use with the compounds of the present invention without undue experimentation.

Pharmaceutical compositions for use in the present method of treatment include those suitable for inhalation, oral, rectal, vaginal, topical (including buccal, dermal and ocular) administration. The compositions may be prepared by any of the methods well known in the art. The most suitable route of administration in any case will depend upon the location of the tissue to be treated, the nature and severity of the condition being treated, and the particular active compound which is being used, as will be apparent to those skilled in the art. The dosage of active compound for treatment of diseases of the respiratory tract will vary depending on the condition being treated and the state of the subject. One skilled in the art would be able to determine appropriate dosages of specific compounds without undue experimentation, using dose response studies as are known in the art.

The active compounds disclosed herein may be administered to the airways of a subject by any suitable means, but are preferably administered by generating an aerosol comprised of respirable particles, the respirable particles comprised of the active compound, which particles the subject inhales. The respirable particles may be liquid or solid. The particles may optionally contain other therapeutic ingredients. (*See, e.g*., US Patent No. 5,849,706 to Molina y Vedia et al.)

In methods of treating the bronchi and/or alveoli, particles comprised of active compound for practicing the present invention should include particles of respirable size: that is, particles of a size sufficiently small to pass through the mouth and larynx upon inhalation and into the bronchi and alveoli of the lungs. In general, particles ranging from about 0.5 to 10 microns in size (more particularly, less than about 5 microns in size) are respirable. Particles of non-respirable size which are included in the aerosol tend to deposit in the throat and be swallowed, and the quantity of non-respirable particles in aerosols intended for treatment of the alveoli and/or bronchi is preferably minimized. For nasal administration, a particle size in the range of 10-500 microns is preferred to ensure retention in the nasal cavity.

Liquid pharmaceutical compositions of active compound for producing an aerosol can be prepared by combining the active compound with a suitable vehicle, such as sterile pyrogen free water.

Administration of the active compounds may be carried out therapeutically or prophylactically (e.g., before substantial lung blockage due to retained mucus secretions has occurred, or at a time when such retained secretions have been at least in part removed, as discussed above.)

Aerosols of liquid particles comprising the active compound may be produced by any suitable means, such as with a nebulizer. See, e.g., U.S. Pat. No. 4,501,729. Nebulizers are commercially available devices which transform solutions or suspensions of the active ingredient into a therapeutic aerosol mist either by means of acceleration of a compressed gas, typically air or oxygen, through a narrow venturi orifice or by means of ultrasonic agitation. Suitable formulations for use in nebulizers consist of the active ingredient in a liquid carrier, typically water or a dilute aqueous alcoholic solution, and preferably made isotonic with body fluids.

Aerosols of solid particles comprising the active compound may likewise be produced with any solid particulate medicament aerosol generator. One illustrative type of solid particulate aerosol generator is an insufflator.

Formulations suitable for oral administration may be presented in discrete units, such as capsules, cachets, lozenges, or tablets, each containing a predetermined amount of the active compound; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients as noted above). Formulations for oral administration may optionally include enteric coatings known in the art to prevent degradation of the formulation in the stomach and provide release of the drug in the small intestine.

Formulations suitable for rectal or vaginal administration may be presented as unit dose suppositories. These may be prepared by admixing the active compound with one or more conventional solid carriers, for example, cocoa butter, and then shaping the resulting mixture.

Formulations suitable for topical application to the eye, mouth, nasal or other surfaces may take the form of an ointment, cream, lotion, paste, gel, spray, aerosol, or oil.

### Peptides

The myristoylated alanine-rich C kinase substrate (MARCKS) protein is a major cellular substrate for protein kinase C. MARCKS is regulated in a cell-, tissue- and developmental stage-specific manner, and expression of MARCKS can be stimulated by various cytokines. MARCKS has been identified in human, bovine, rodent and avian species. Harlan et al., J.Biol. Chem. 266:14399 (1991); Graff et al., J. Biol. Chem. 266:14390 (1991); Graff et al., Mol. Endocrinol. 3:1903 (1989); Stumpo et al., Proc. Natl. Acad. Sci. USA 86:4012-16 (June 1989).

Peptides corresponding to the MARCKS protein are commercially available. A MARCKS "psd peptide" is available from BIOMOL (Plymouth Meeting, Pennsylvania), having the sequence KKKKKRFSFK KSFKLSGFSF KKNKK (SEQ ID NO:2). *See* P. Blackshear, J. Biol. Chem. 268:1501 (1993).

The present inventors have identified two specific active fragments of MARCKS protein that are able to affect mucus secretion. A myristoylated polypeptide, 24 amino acids in length, with sequence Myristic acid-GAQFSKTAAKGEAAAERPGEAAVA (SEQ ID NO:1), is referred to herein as the MANS peptide for myristolated N-terminal sequence. The peptide inhibits secretion of mucus from mucous membranes and mucus-secreting cells, including human airway epithelial cells. The present inventors' data suggests that this peptide blocks the attachment of MARCKS protein to the mucin granule, thus blocking or inhibiting the release of mucin granules and the secretion of mucus by the cell.

A second peptide corresponding to the PSD (phosphorylation) site of MARCKS was also tested. At some concentrations this peptide stimulates mucus secretion, while at other doses (higher) it has no effect on stimulated secretion (**Figure 3**), and it is predicted that even higher doses will decrease stimulated mucus secretion. The PSD peptide sequence is myristic acid-KKKKKRFSFKKSFKLSGFSFKKNKK (SEQ ID NO:2), referred to herein as the MA-PSD peptide. While not wishing to be held to a single theory underlying the present invention, the inventors believe that MARCKS protein fragments that are able to increase mucus secretion (such as the MA-PSD peptide, SEQ ID NO:2) may be binding to endogenous proteins in the cell that competitively inhibit the phosphorylation of MARCKS, thus inhibiting the release of MARCKS from the plasma membrane into the cell interior (see **Figure 4**). One such inhibitor of MARCKS phosphorylation is calmodulin. Other "MARCKS inhibitors", for purposes of the present invention, are those endogenous compounds that prevent the MARCKS protein from completing a necessary step in the mucus-secretion pathway. MARCKS inhibitors may thus act to inhibit the phosphorylation or the dephosphorylation of MARCKS (each of which is necessary in the present pathway), or bind to MARCKS to prevent its binding to the mucin granule membrane. Compounds of the present invention that increase the secretion of mucus may be acting by binding to such endogenous inhibitors, thus freeing endogenous MARCKS protein to complete the mucus-secretion pathway.

Thus, peptide fragments of the MARCKS protein may be designed, tested and selected for their ability to inhibit or enhance mucus secretion, using the present disclosure and methods known in the art.

The nucleotide and amino acid sequences of human MARCKS cDNA and protein as reported by Harlan et al., J. Biol. Chem. 266:14399 (1991) (GenBank Accession No. M68956) are provided as SEQ ID NO:3 and SEQ ID NO:4. The nucleotide and amino acid sequences of human MARCKS cDNA and protein as reported by Sakai et al., Genomics 14:175 (1992) are provided as SEQ ID NO:5 and SEQ ID NO:6. An additional publication (Harlan et al., J. Biol. Chem. 266(22):14399 (1991) provides a nucleotide sequence for human MARCKS that differs from that of Sakai et al. at nucleotides 619 and 724; in this sequence, G is substituted for T at position 619 and C is substituted for G at position 724. Additional allelic variants of human and other MARCKS proteins would be expected.

While not wishing to be held to a single theory underlying the present invention, the present inventors propose that the pathway for the involvement of MARCKS in mucus secretion in airway epithelium is as shown in **Figure 4**. It is currently believed that active peptide fragments of MARCKS affect mucus secretion at the level of the interaction of MARCKS with the mucin granules, which contain the major protein components of mucus. As shown in **Figure 4**, the present inventors believe that MARCKS must be dephosphorylated to bind to the mucin granule, which triggers mucin exocytosis and results in mucus secretion.

The methods of the present invention include the use of isolated DNA molecules encoding the peptides of the present invention. Such isolated DNA molecules are useful in producing the therapeutic peptides, and may additionally be used in an appropriate gene expression vector for gene therapy, using methods as are known in the art for the expression of the peptide *in vivo.* Cell-specific or inducible promoters may further be used to control the expression of the therapeutic peptide *in vivo.* Methods of delivering DNA encoding a desired peptide to achieve a therapeutic effect is disclosed, e.g. in US Patent No. 5,580,859 and 5,703,055 to Felgner et al.

Analogs of the therapeutic peptides disclosed herein are described . As used herein, an "analog" is a chemical compound similar in structure to a first compound, and having a similar physiologic action as the first compound. With particular reference to the present invention, MARCKS peptide analogs are those compounds which, while not having the exact amino acid sequences of the native MARCKS fragment, are capable of binding to the same sites as the native MARCKS fragment. Such analogs may be peptide or non-peptide analogs, including nucleic acid analogs, as described in further detail below.

In protein molecules which interact with a receptor, the interaction between the protein and the receptor must take place at surface-accessible sites in a stable three-dimensional molecule. By arranging the critical binding site residues in an appropriate conformation, peptides which mimic the essential surface features of the p20 ligands may be designed and synthesized in accordance with known techniques.

Methods for determining peptide three-dimensional structure and analogs thereto are known, and are sometimes referred to as "rational drug design techniques". See, e.g., U.S. Patent No. 4,833,092 to Geysen; U.S. Patent No. 4,859,765 to Nestor; U.S. Patent No. 4,853,871 to Pantoliano; U.S. Patent No. 4,863,857 to Blalock; See *also* Waldrop, Science, 247, 28029 (1990); Rossmann, Nature, 333, 392-393 (1988); Weis et al., Nature, 333, 426-431 (1988); James et al., Science, 260, 1937 (1993) (development of benzodiazepine peptidomimetic compounds based on the structure and function of tetrapeptide ligands).

In general, those skilled in the art will appreciate that minor deletions or substitutions may be made to the amino acid sequences of peptides of the present invention without unduly adversely affecting the activity thereof. Thus, peptides containing such deletions or substitutions are also described. In peptides containing substitutions or replacements of amino acids, one or more amino acids of a peptide sequence may be replaced by one or more other amino acids wherein such replacement does not affect the function of that sequence. Such changes can be guided by known similarities between amino acids in physical features such as charge density, hydrophobicity/hydrophilicity, size and configuration, so that amino acids are substituted with other amino acids having essentially the same functional properties. For example: Ala may be replaced with Val or Ser; Val may be replaced with Ala, Leu, Met, or Tie, preferably Ala or Leu; Leu may be replaced with Ala, Val or Ile, preferably Val or Ile; Gly may be replaced with Pro or Cys, preferably Pro; Pro may be replaced with Gly, Cys, Ser, or Met, preferably Gly, Cys, or Ser; Cys may be replaced with Gly, Pro, Ser, or Met, preferably Pro or Met; Met may be replaced with Pro or Cys, preferably Cys; His may be replaced with Phe or Gln, preferably Phe; Phe may be replaced with His, Tyr, or Trp, preferably His or Tyr; Tyr may be replaced with His, Phe or Trp, preferably Phe or Trp; Trp may be replaced with Phe or Tyr, preferably Tyr; Asn may be replaced with Gln or Ser, preferably Gln; Gln may be replaced with His, Lys, Glu, Asn, or Ser, preferably Asn or Ser; Ser may be replaced with Gln, Thr, Pro, Cys or Ala; Thr may be replaced with Gln or Ser, preferably Ser; Lys may be replaced with Gln or Arg; Arg may be replaced with Lys, Asp or Glu, preferably Lys or Asp; Asp may be replaced with Lys, Arg, or Glu, preferably Arg or Glu; and Glu may be replaced with Arg or Asp, preferably Asp. Once made, changes can be routinely screened to determine their effects on function with enzymes.

Non-peptide mimetics of the peptides of the present invention are also described. Non-protein drug design may be carried out using computer graphic modeling to design non-peptide, organic molecules which bind to sites bound by the native MARCKS fragments disclosed herein. See, e.g., Knight, BIO/Technology, 8, 105 (1990). Itzstein et al, Nature, 363, 418 (1993); Lam et al, Science, 263, 380 (Jan. 1994) (rational design of bioavailable nonpeptide cyclic ureas that function as HIV protease inhibitors). Analogs may also be developed by generating a library of molecules, selecting for those molecules which act as ligands for a specified target, and identifying and amplifying the selected ligands. See, e.g., Kohl et al., Science, 260, 1934 (1993). Techniques for constructing and screening combinatorial libraries of oligomeric biomolecules to identify those that specifically bind to a given receptor protein are known. Suitable oligomers include peptides, oligonucleotides, carbohydrates, non-oligonucleotides (e.g., phosphorothioate oligonucleotides; see Chein. and Engineering News, page 20, 7 Feb. 1994) and nonpeptide polymers (see, e.g., "peptoids" of Simon et al., Proc. Natl. Acad. Sd. USA, 89, 9367 (1992)). See *also* U.S. Patent No. 5,270,170 to Schatz; Scott and Smith, Science, 249, 386-390 (1990); Devlin et al., Science 249, 404-406 (1990); Edgington, BIO/Technology, 11, 285 (1993). Peptide libraries may be synthesized on solid supports, or expressed on the surface of bacteriophage viruses (phage display libraries). Known screening methods may be used by those skilled in the art to screen combinatorial libraries to identify suitable peptide analogs. Techniques are known in the art for screening synthesized molecules to select those with the desired activity, and for labelling the members of the library so that selected active molecules may be identified. See, e.g., Brenner and Lerner, Proc. Natl. Acad. Sci. USA, 89, 5381 (1992); PCT U593/06948 to Berger et al.,; Simon et al., Proc. Natl. Acad. Sci. USA, 89, 9367, (1992); U.S. Patent No. 5,283,173 to Fields et al.

As used herein, "combinatorial library" refers to collections of diverse oligomeric biomolecules of differing sequence, which can be screened simultaneously for activity as a ligand for a particular target. Combinatorial libraries may also be referred to as "shape libraries", i.e., a population of randomized polymers which are potential ligands. The shape of a molecule refers to those features of a molecule that govern its interactions with other molecules, including Van der Waals, hydrophobic, electrostatic and dynamic.

Nucleic acid molecules may also act as ligands for receptor proteins. See, e.g., Edgington, BIO/Technology, 11, 285 (1993). U.S. Patent No. 5,270,163 to Gold and Tuerk describes a method for identifying nucleic acid ligands for a given target molecule by selecting, from a library of RNA molecules with randomized sequences, those molecules that bind specifically to the target molecule. A method for the *in vitro* selection of RNA molecules immunologically cross-reactive with a specific peptide is disclosed in Tsai, Kenan and Keene, Proc. Natl. Acad. Sci. USA, 89, 8864 (1992) and Tsai and Keene, J. Immunology, 150, 1137 (1993).

### Antisense Oligonucleotides

The present inventors have further demonstrated that antisense oligonucleotides directed against MARCKS mRNA decreases (inhibits) mucus secretion in human airway epithelial cells. (*See* Example 6 and **Figure 5**).

It has been demonstrated that antisense oligonucleotides that are complementary to specific RNAs can inhibit the expression of cellular genes as proteins. *See* Erickson and Izant, Gene Regulation: Biology Of Antisense RNA And DNA, Vol. 1, Raven Press, New York, 1992. For example, selective inhibition of a p21 gene that differed from a normal gene by a single nucleotide has been reported. Chang et al., Biochemistry 1991,30:8283-8286. Many hypotheses have been proposed to explain the mechanisms by which antisense oligonucleotides inhibit gene expression, however, the specific mechanism involved may depend on the cell type studied, the RNA targeted, the specific site on the RNA targeted, and the chemical nature of the oligonucleotide. Chiang et al., J. Biol. Chem. 1991, 266:18162-18171; Stein and Cohen, Cancer Res. 1988, 48:2659-2668.

Described are oligonucleotides substantially complementary to a MARCKS protein nucleotide sequence that occurs endogenously in a mucus-secreting cell. Such oligonucleotides are useful in decreasing mucus production by cells into which they are delivered. "Nucleotide sequence" refers to a polynucleotide formed from a series of joined nucleotide units. The term "substantially complementary", as used herein, refers to that amount of sequence complementarity between the oligonucleotide and a MARCKS gene nucleotide sequence which allows for interstrand hybridization under physiological conditions and enables the oligonucleotide to inhibit the expression of the MARCKS gene. Interstrand hybridization is the interaction between the oligonucleotide and the MARCKS nucleotide sequence. The potential of forming a stable interstrand hybrid can be determined by those skilled in the art using methods known in the art, such as, for example, determination of the melting temperature for the hybrid by mathematical modeling or empirical analysis, or solid support nucleic acid hybridizations. (*See, e.g*., Marmur and Doty, J. Mol. Biol. 1962, 5, 113).

Antisense DNAs used are able to produce the corresponding antisense RNAs. An antisense RNA molecule has the nucleotide bases in the reverse or opposite order for expression. Such antisense RNAs are well known in the art, see e.g., US Patent No. 4,801,540 to Calgene Inc.

As used herein, the term "MARCKS nucleotide sequence" refers to any nucleotide sequence derived from a gene encoding a MARCKS protein, including, for example, DNA or RNA sequence, DNA sequence of the gene, any transcribed RNA sequence, RNA sequence of the pre-mRNA or mRNA transcript, and DNA or RNA bound to protein.

Oligonucleotides targeted to sequences in MARCKS genes can be used to inhibit mucus production in epithelial cells. The oligonucleotide may be any length of sequence capable of forming a stable hybrid with the endogenous MARCKS nucleotide sequence under physiologic conditions. It is preferred that the length of the oligonucleotide be between 5 and 200 nucleotides. It is more preferred that the oligonucleotide be between 10 and 50 nucleotides in length. It is most preferred that the oligonucleotide be between 15 and 25 nucleotides in length.

The nucleotides of the oligonucleotides may be any known in the art including natural and synthetic moieties. The term "oligonucleotide" as used herein refers to a polynucleotide formed from joined nucleotides. Moreover, the term "oligonucleotide" includes naturally occurring oligonucleotides or synthetic oligonucleotides formed from naturally occurring subunits or analogous subunits designed to confer special properties on the oligonucleotide so that it is more stable in biological systems or binds more tightly to target sequences. It also includes modifications of the oligonucleotides such as chemically linking them to other compounds that will enhance delivery to cells or to the nucleus and other compartments of cells. Oligonucleotides of the invention may be synthesized by any method known in the art, including synthetic chemical methods. *See, e*.*g*., Vu and Hirschbein, Tetrahedron Lett. 1991, 32:30005-30008. Oligonucleotides may be modified via chemical methods known to those skilled in the art, including encapsulation in liposomes, or chemical linkage to steroids, antibodies, and cell receptors.

Described is an oligonucleotide complementary to an endogenous MARCKS nucleotide sequence found in the cell to be treated, or having sufficient complementarity to allow stable interstrand hybridization between the oligonucleotide and an endogenous MARCKS nucleotide, and that inhibits the expression of the MARCKS gene. A preferred oligonucleotide is one that is complementary to a MARCKS nucleotide sequence derived or selected from a mammal, in particular, a human.

The oligonucleotides may be oligodeoxyribonucleotides or oligoribonucleotides, including modified oligodeoxynucleotides and oligoribonucleotides. Moreover, the oligonucleotides may be comprised of combinations of deoxyribonucleotides and ribonucleotides. Further, oligonucleotides may also include modified subunits. For example, phosphorothioate oligodeoxyribonucleotides may be included. It is preferred that the oligonucleotides be modified to increase stability and prevent intracellular and extracellular degradation. It is more preferred that the oligonucleotides be modified to increase their affinity for target sequences, and their transport to the appropriate cells and cell compartments when they are delivered into a mammal in a pharmaceutically active form.

It is preferred that the oligonucleotides be antisense oligonucleotides. The oligonucleotides may be targeted to a noncoding portion of a MARCKS or targeted to coding sequences of the gene, and may include an intron-exon junction (i.e., several nucleotides on either or both sides of the intron-exon junction).

The oligonucleotides may be administered by any method that produces contact of the oligonucleotide with the target tissue or cell in the subject being treated, including but not limited to oral administration, topical administration, and inhalation. The pharmaceutical compositions comprising the oligonucleotides may be in solid dosage forms, such as capsules, tablets, and powders, or in liquid dosage forms, such as elixirs, syrups, and suspensions. The dosage administered varies depending upon factors such as: pharmacodynamic characteristics; its mode and route of administration; age, health, and weight of the recipient; nature and extent of symptoms; kind of concurrent treatment; and frequency of treatment and the effect desired. Effective dosages are those which are able to inhibit mucus production in the airways at a level which alleviates, reduces, or eliminates the symptoms or conditions associated with the mucus production.

The oligonucleotides may be administered singly, or in combination with other compounds, other pharmaceutical compounds, or therapies. The oligonucleotides are preferably administered with a pharmaceutically acceptable carrier or diluent selected on the basis of the selected route of administration and standard pharmaceutical practice.

Inhibition of secretion of mucus, via inhibition of MARCKS protein function in epithelial secretory cells, is a focus of this invention. To achieve this end, the invention describes methods of inhibiting mucus secretion which comprises contacting a mucus-secretory cell with a MARCKS gene expression inhibitory amount of an oligonucleotide substantially complementary to an endogenous MARCKS gene nucleotide sequence. The invention also includes a method whereby the contacting step comprises lipofectin as a carrier for the oligonucleotide. The oligonucleotides of the invention are administered to mammals or avians, and preferably to humans, in therapeutically effective amounts or concentrations which are effective to inhibit or reduce mucus production in the target tissue or organ.

The oligonucleotides will be capable of reaching their intracellular target to inhibit or reduce the expression of MARCKS protein therein. The invention therefore provides methods of inhibiting mucus secretion which comprise contacting at least one element of MARCKS gene expression machinery with a gene expression inhibitory amount of an oligonucleotide. For the purposes of the invention, the elements of the gene expression machinery may comprise any nucleotide sequence of a MARCKS gene, the nucleotide sequence of spliced mRNAs transcribed from a gene, unspliced RNAs and partially spliced RNAs transcribed from a gene, DNA-RNA hybrids comprising sequence derived from a gene, such as in actively transcribing genes, RNA transcribed from a gene bound to protein, and any molecule or structure known in the art to be involved in gene expression.

US Patent No. 5,858,784 to Debs et al. provides a method of administering nucleic acids to the lung cells of a subject by preparing a liposome-nucleic acid mixture suitable for nebulization, nebulizing the mixture, and depositing the resulting nebulized mixture in the lungs of the subject. The nucleic acid sequence may include DNA sequences which encode polypeptides which are directly or indirectly responsible for a therapeutic effect, or active nucleotide sequences such as antisense sequences and ribozymes. The nucleic acid constructs can be provided to the cells of the subject as expression cassettes; preferably, the construct does not become integrated into the host cell genome and is introduced into the host as part of a non-integrating expression vector.

### Double Stranded RNA and Ribozymes

It has recently been shown that the introduction of exogenous doublestranded RNA (dsRNA) can specifically disrupt the activity of genes containing homologous sequences, possibly by post-transcriptional effects. Montgomery et al., Proc. Natl. Acad. Sci. USA 95:15502 (1998); Ngo et al., Proc. Natl. Acad. Sci. USA 95:14687 (1998). Accordingly, the methods described herein may be carried out by introducing exogenous dsRNA into a mucus-secreting cell, where the dsRNA has sufficient sequence similarity to the RNA of an endogenous MARCKS gene to result in a reduction in MARCKS protein in the cell (compared to that which would occur in the absence of the exogenous dsRNA).

The administration of dsRNA may be carried out using the methods discussed above regarding peptide and antisense oligonucleotide administration.

In an alternate embodiment, DNA encoding an enzymatic RNA molecule (ribozyme) may be introduced into the target cell. Ribozymes are directed against and cleave the mRNA transcript of the cell's endogenous MARCKS protein. DNA encoding enzymatic RNA molecules may be produced in accordance with known techniques (see e.g., US Patent No. 4,987,071. Production of such an enzymatic RNA molecule and disruption of MARCKS protein production affects mucus production by the target cell in essentially the same manner as production of an antisense RNA molecule.

### Methods of Screening

Also described is a method of screening compounds for their ability to affect (enhance or inhibit) mucus production. Combinatorial chemistry processes as are known in the art may be used to generate large numbers of structurally diverse compounds, which can then be screened. Such screening methods comprise providing a culture of mucus-secreting cells, such as the cultures of normal human bronchial epithelial cells described in Example 1 herein. A test compound is administered to the cells, and the cells may also be exposed to a compound known to stimulate mucus production (e.g., PMA, UTP, 8-bromo-cGMP). The test compound and the stimulatory compound may be administered to the cells, for example, by exposing the cells to media containing the compounds. The cells may, for example, be pre-incubated with the test compound first, then co-incubated with the stimulatory compound and the test compound. Alternatively, the pre-incubation step may be omitted. The ability of the test compound to bind to either the mucin granule membrane (or a mucin granule membrane-related receptor) or to endogenous MARCKS protein at the mucin granule membrane binding site is assessed by detecting whether the test compound inhibits binding of endogenous MARCKS to the mucin granule. Such detection can be carried out using methods known in the art, for example, by labelling the test compound with a detectable molecule.

Molecules detectable by spectroscopic, photochemical, biochemical, immunochemical, electrical and optical means are known. Optically detectable molecules include fluorescent labels (fluorescein, Texas Red, Green Fluorescent Protein). Methods for viewing intact cells are known, including real-time confocal laser-scanning microscopy and two-photon laser-scanning microscopy.

Mucus secreted by the cells may also be measured after a pre-determined time period, for example using an ELISA assay as is known in the art. The mucus secretion of the cells exposed to the test compound can also be compared to that of control cells that were not exposed to the test compound. A decrease in mucus secretion by the test cells compared to the control cells indicates that the test compound inhibits mucus secretion, and an increase in mucus secretion by the test cells compared to the control cells indicates that the test compound enhances mucus secretion.

The following examples are provided to more fully illustrate the present invention and should not be construed as limiting thereof.

### EXAMPLE 1

### In vitro Assessment of Mucus Secretion

**Cell Culture System:** *Expansion and cryopreservation.* Primary normal human bronchial epithelial (NHBE) cells (Clonetics, San Diego, CA) were seeded into vented T75 tissue culture flasks (500 cells/cm²) in bronchial epithelial basal medium (BEBM; Clonetics, San Diego, CA) containing 25 ng/ml human recombinant epidermal growth factor (EGF; Intergen, Purchase, NY), 65 ng/ml bovine pituitary extract (prepared by the methods of Bertolero et al. Exp Cell Res 155:64, 1984), 5 x 10⁻⁸M all-trans retinoic acid, 1.5µg/ml bovine serum albumin (Intergen, Purchase, NY), 20 IU/ml nystatin (Gibco, Grand Island, NY), 0.5µg/ml hydrocortisone, 5µg/ml insulin, 10µg/ml transferrin, 0.5µg/ml epinephrine, 6.5ng/ml triiodothyronine, 50µg/ml gentamicin, and 50µg/ml amphotericin-B (Clonetics; San Diego, CA). Once confluent, cultures were dissociated with trypsin/EDTA and frozen as passage-2 according to the methods of Clonetics Corporation.

*Air-liquid interface culture of NHBE cells.* Following the expansion, NHBE cells were cultured in air/liquid interface according to the methods of Gray and coworkers with minor modifications (Gray et al. Am J Respir Cell Mol Biol 14:104, 1996). The air-liquid interface culture was initiated by seeding NHBE cells (passage-2, 2x10⁴ cells/cm²) on Transwell-clear culture inserts (24.5 mm, 0.45 µm pore size; Costar, Cambridge, MA) that were thin coated with rat tail collagen, type I (Collaborative Research, Bedford, MA). Cells were cultured submerged to 70% confluency (5-7 days) in a 1:1 mixture of bronchial epithelial cell growth medium (Clonetics, San Diego, CA):Dulbecco's modified Eagles medium with high glucose (BEGM:DMEM-H), containing the same supplements as described above with the exception of EGF (0.5 ng/ml). When cultures were 70% confluent, the air-liquid interface was created by removing the apical medium, and the basal medium (BEGM:DMEM-H) was changed daily thereafter. Cells were then cultured for an additional 14 days in air-liquid interface, a total of 21 days in culture.

**Mucus ELISA:** Mucus secreted from the airway epithelial cells in vitro after stimulation by activators was assessed using an ELISA (antibody capture method) (E. Harlow, D. Lane. "Antibodies: A Laboratory Manual." New York: Cold Spring Harbor Laboratory Press, 1988), wherein the collected mucus is bound directly to the ELISA plate. Mucus was detected using an antibody raised against monkey airway mucus (Lin et al. Am J Respir Cell Mol Biol 1:41, 1989).

### EXAMPLE 2

### MARCKS mRNA in Human Bronchial Epithelial Cells

MARCKS messenger RNA was detected in human bronchial epithelial cells grown in air/liquid interface culture by Northern analysis (Ausubel et al., eds. "Current Protocols in Molecular Biology." New York: John Wiley & Sons, 1992) using a human MARCKS cDNA (approximate length 1 kb) as a radiolabelled probe. MARCKS message increases as these cells become more differentiated when maintained in an air/liquid interface culture.

To detect MARCKS protein in these cells, cells were labeled with ³H-myristic acid (as MARCKS is myristoylated) for 16 hours in media. Cells were lysed, and MARCKS protein was immunoprecipitated according to the method of Spizz & Blackshear (J Biol Chem 271:553, 1996) using monoclonal antibody 2F12 (a gift from the Blackshear laboratory).

MARCKS within the airway epithelial cells was found to be phosphorylated by the PKC activator, PMA (100 nM), while 4α-PMA (a phorbol ester control which does not activate PKC), did not phosphorylate MARCKS. Phosphorylation of MARCKS by PMA was attenuated by Calphostin C (500 nM). NHBE cells also contained substantial amounts of cGMP-dependent protein kinase type 1α (PKG-1α) activity, which was localized to the cytosolic fraction. The cells exhibited constitutive PKG activity which was increased by incubation with 100 µM dibutyryl cGMP. In addition, the phosphorylation of MARCKS induced by PMA was reversed by incubation with 8-Br-cGMP (10 µM). Okadaic acid (500 nM) inhibited this effect. These results indicate that 8-Br-cGMP activates a phosphatase (type 1 or 2A), which dephosphorylates MARCKS.

### EXAMPLE 3

### Blocking of Mucin Secretion by Peptide MANS

The effect on mucus secretion of a myristoylated peptide containing the first 24 amino acids of the human MARCKS protein (MANS; myristoylated N-terminal sequence; SEQ ID NO:1) was tested. Cultured normal human bronchial epithelial cells as described above were used. Test cells were co-incubated for 15 minutes in apical and basolateral media containing 1, 10 or 100 µM of MANS peptide, and then co-incubated for 15 minutes with the peptide and 100 nM PMA plus 1 µM 8-Br-cGMP (columns 3-5 of **Figure 1A**). Control cells were not exposed to MANS peptide but were preincubated in media only (column 1 of **Figure 1A**) or media containing PMA and 8-Br-cGMP (column 2 of **Fig. 1A**). Single asterisks (*) indicate that the response was statistically different than the media control (column 1), and double asterisks (**) indicate that the response was statistically different than that observed in stimulated cells that were not exposed to the MANS peptide (column 2).

Stimulation by PMA and 8-Br-cGMP caused at least a 100% increase in mucus secretion over control levels. This increase, however, was blocked by pre-and co-incubation with the MANS peptide. Levels of secreted mucus fell to control values when 10 µM peptide was used, and levels of secreted mucus were well below control values following incubation with 100 µM MANS peptide (**Figure 1A**).

The MANS peptide (100µM) was also found to decrease constitutive (basal) mucus secretion by one hour incubation. Cells were treated as described above except that no PMA or 8-Br-cGMP was used. In addition, a negative control peptide of the same amino acid composition as the MANS peptide in random order (RNS; random N-terminal sequence) did not affect constitutive mucus secretion. Results are graphed in **Figure 1B**. Single asterisk (*) indicates that the response was statistically different than the media control (column 1).

### EXAMPLE 4

### UTP-induced Mucin Secretion is Blocked by Peptide MANS

These experiments were carried out similarly to those described in Example 3. To test for stimulated secretion, the cells were exposed apically and basolaterally to uridine 5'-triphosphate (UTP) at a concentration of 0.1 mM in media. Cells were pre-incubated for 15 minutes with the MANS peptide and test cultures were then co-incubated with the MANS peptide and UTP for 45 minutes.

Results are shown in **Figure 2**, where column 1 is the media/control; column 2 = 0.1 mM UTP; column 3 = 0.1 mM UTP and 1 µM MANS peptide; column 4 = 0.1 mM UTP and 10 µM MANS peptide; and column 5 = 0.1 mM UTP and 100 µM MANS peptide. Single asterisks (*) indicate that the measured response was statistically different than the media control (column 1), and double asterisks (**) indicate that the response was statistically different than that observed in stimulated cells that were not exposed to the MANS peptide (column 2). The MANS peptide at 10 and 100 µM significantly reduced UTP-stimulated mucus secretion.

### EXAMPLE 5

### Effect of MA-PSD Peptide on Mucin Secretion

These experiments were carried out using normal human bronchial epithelial cells *in vitro* as described above. A myristoylated peptide composed of the 25 amino acid phosphorylation site domain of MARCKS (MA-PSD peptide; SEQ ID NO:2) was prepared. Test cells were preincubated for 15 minutes in apical and basolateral media containing the MA-PSD peptide (1, 10 or 100 µM), and then co-incubated for 15 minutes with the peptide and stimulus (100 nM PMA plus 1 µM 8-Br-cGMP). Control cells were preincubated in media only (column 1 of **Figure 3A**); or with 100 nM PMA only (column 2); or with 100 nM PMA and 1 µM 8-Br-cGMP (column 3).

Stimulation by PMA and 8-Br-cGMP caused about a 100% increase in mucus secretion over control levels. This increase was augmented in a dose-dependent manner by pre- and co-incubation with the MA-PSD peptide, 1 or 10 µM. Interestingly, stimulated levels of mucus secretion were unaffected by the presence of 100 µM peptide. Results are graphed in **Figure 3A**. Results using MANS peptide (1, 10 and 100 µM) are provided in columns 7-9 for comparison. Single asterisks (*) indicate that the measured response was statistically different than the media control (column 1), and double asterisks (**) indicate that the response was statistically different than that observed in stimulated cells that were not exposed to the peptide (column 3).

The effect of MA-PSD peptide (1, 10 and 100µM) on basal mucin secretion was also measured. Cells as described above were incubated for one hour with the MA-PSD peptide (no PMA or 8-Br-cGMP). Results are graphed in **Figure 3B**. The single asterisk (*) indicates that the response to 100 µM of MA-PSD peptide was statistically different than the media control (column 1), whereas no statistically significant difference was seen when 1 or 10 µM of peptide was used (columns 3 and 4).

### EXAMPLE 6

### Inhibition of mucus secretion by antisense oligonucleotides

Using an antisense oligonucleotide directed to the endogenous human MARCKS gene, mucus secretion was inhibited *in vitro* in human airway epithelial cells. The *in vitro* assay system as described in Example 1 was utilized to test the effects of antisense oligonucleotides to MARCKS mRNA.

An antisense oligonucleotide was constructed by a commercial supplier (Chemicon International, Temecula, CA; in conjunction with Biognostik GmbH, Gottingen, Germany) based on the human MARCKS gene sequence of Sakai et al. (Genomics 14:175 (1992); GenBank accession number D10522, D90498). A control oligonucleotide was also constructed.

The oligonucleotides were administered to the differentiated airway epithelial cultures by incubation in media containing the oligonucleotides (5 µM) for three days. The oligonucleotide was supplied to the apical surface of the cells in 0.4 ml of media containing lipofectin reagent (2 µg/ml; Gibco BRL). Cells were incubated with the oligonucleotide in the presence of lipofectin for 24 hours. Following a media change, cells were incubated with the oligonucleotide alone for an additional 48 hours. To test the ability of the oligonucleotides to affect stimulated mucus secretion, test cells were stimulated for 15 minutes with 100 nM PMA and 1 µM 8-Br-cGMP (activators of PKC and PKG, respectively).

Results are shown in **Figure 5**, where column 1 = media/control; column 2 = cells stimulated with PMA and 8-Br-cGMP; column 3 = cells exposed to 5µM control oligonucleotide and stimulated with PMA and 8-Br-cGMP; and column 4 = cells exposed to 5µM antisense oligonucleotide and stimulated with PMA and 8-Br-cGMP. Single asterisks (*) indicate that the measured response was statistically different than the media control (column 1), and double asterisks (**) indicate that the response was statistically different than that observed in stimulated cells that were not exposed to an oligonucleotide (column 2).

Mucus secreted from the airway epithelial cells after stimulation by PKC and PKG activators was assessed using an ELISA (antibody capture method). The control oligonucleotide (column 3) had no effect on stimulated mucus secretion. In contrast, the antisense oligonucleotide (column 4) caused a statistically significant decrease in mucus secretion compared to stimulated levels.

These results indicate that MARCKS antisense oligonucleotides inhibit stimulated mucus secretion, although a basal level of mucus secretion can be maintained by selection of appropriate dosages. In contrast, control oligonucleotides had no effect on stimulated secretion.

### EXAMPLE 7

### TNF-α Up-Regulates MARCKS Expression And Augments Mucin Hypersecretion

NHBE cells were incubated with 10 ng/ml human recombinant TNF-α or medium alone for 4 hrs, then stimulated with PMA (100nM) + 8-Br-cGMP (1 µm) for 15 min, or UTP (0.1 mM) for 2 hrs. Secreted mucin was collected and measured by ELISA. Total RNA and protein were isolated from treated cells. MARCKS mRNA was assessed by Northern hybridization, and protein by Western blotting. The results are shown in **Figures 6A, 6B** and **6C**.

The Northern Blot and graph shown in **Figure 6A** show an increase in MARCKS mRNA in cells incubated with TNF-α (lane 2 of blot, column 2 of graph) compared to cells incubated in medium alone (lane 1 of blot, column 1 of graph). The Western-blot and graph shown in **Figure 6B** show a three- to four-fold increase in MARCKS protein in cells incubated with TNF-α (lane 2 of blot, column 2 of graph) as compared with cells incubated with medium only (lane 1 of blot, column 1 of graph). The graph in **Figure 6C** shows that in cells incubated with TNF-α, mucin hypersecretion was significantly augmented in response to subsequent stimulation by PMA + 8-Br-cGMP or UTP when compared to mucin secretion of cells incubated in medium only. Data are presented as mean ± SEM (n=6 at each point). Single asterisks (*) indicate a statistically significant difference from control (medium-only) samples (*p*<0.05). Single cross marks ( †) indicate a statistically significant difference from stimulus (*p*<0.05).

### EXAMPLE 8

### Okadaic acid blocks stimulated mucin hypersecretion

NHBE cells were pre-incubated with okadaic acid (500nM) for 15 min at 37°C/5%CO₂, then stimulated with PMA (100 nM) + 8-Br-cGMP (1µM) for 15 min, or with UTP (0.1 mM) for 2 hours. Secreted mucin in the apical medium was collected and assayed by ELISA. The results are shown in **Figure 7**.

The graph shown in **Figure 7** shows that okadaic acid, a phosphatase inhibitor, blocks mucin hypersecretion induced by PMA + 8-Br-cGMP or UTP. Column 1 shows the results of incubation with medium alone for 30 min. Column 2 shows the results of pre-incubation with medium alone for 15 min, then incubation with PMA + 8-Br-cGMP for an additional 15 min. Column 3 shows the results of pre-incubation with okadaic acid for 15 min, then co-incubation with PMA + 8-Br-cGMP for an additional 15 min. Column 4 shows the results of incubation with medium alone for 2 hrs. Column 5 shows the results of pre-incubation with medium alone for 15 min, then incubation with UTP for an additional 2 hrs. Column 6 shows the results of pre-incubation with okadaic acid for 15 min, then co-incubation with UTP for an additional 2 hrs. Data are presented as mean ± SEM (n=6 at each point). Single asterisks (*) indicate a statistically significant difference from control (*p*<0.05). Single cross marks (†) indicate statistically significant difference from stimulus (*p*<0.05).

### EXAMPLE 9

### Mucin Hypersecretion Induced By UTP Is Inhibited By Inhibitors Of The Mucus Secretion Signaling Pathway

NHBE cells were pre-incubated with the indicated inhibitor for 15 min, then stimulated with UTP (0.1mM) for 2 hours. Secreted mucin in the apical medium was collected and assayed by ELISA. The results are shown in **Figure 8**.

The graph of **Figure 8** shows that mucin hypersecretion induced by UTP involves activation of PKC and PKG. Column 1 indicates the results of incubation with medium alone. Column 2 indicates the results of incubation in 0.1 mM UTP. Column 3 indicates the results of incubation in 0.1mM UTP + 500 nM calphostin C (an inhibitor of PKC). Column 4 indicates the results of incubation with 0.1 mM UTP + 10 µM Rp-8-Br-PET-cGMP (an inhibitor of PKG). Column 5 indicates the results of incubation with 0.1 mM UTP + 50 µM LY83583 (an inhibitor of soluble guanylyl cyclase). Column 6 indicates the results of incubation with 0.1 mM UTP + 500 nM KT5720 (an inhibitor of PKA). Data are presented as mean ± SEM (n=6 at each point). Single asterisks (*) indicate a statistically significant difference from control (*p*<0.05). Single cross marks (†) indicate a statistically significant difference from UTP stimulation (*p*<0.05).

The foregoing is illustrative of the present invention, and is not to be construed as limiting thereof. The invention is defined by the following claims, with equivalents of the claims to be included therein.

### SEQUENCE LISTING

<210> 1
   <211> 24
   <212> PRT
   <213> Homo sapiens
<400> 1 <210> 2 <211> 25 <212> PRT <213> Homo sapiens <400> 2
<210> 3
   <211> 1885
   <212> DNA
   <213> Homo sapiens
<220>
   <221> CDS
   <222> (309)..(1307)
<400> 3
<210> 4
   <211> 332
   <212> PRT
   <213> Homo sapiens
<400> 4
   Met Gly Ala Gln Phe Ser Lys Thr Ala Ala Lys Gly Glu Ala Ala Ala
    1               5                  10                  15
   Glu Arg Pro Gly Glu Ala Ala Val Ala Ser Ser Pro Ser Lys Ala Asn
               20                  25                  30
   Gly Gln Glu Asn Gly His Val Lys Val Asn Gly Asp Ala Ser Pro Ala
           35                  40                  45
   Ala Ala Glu Ser Gly Ala Lys Glu Glu Leu Gln Ala Asn Gly Ser Ala
       50                  55                  60
   Pro Ala Ala Asp Lys Glu Glu Pro Ala Ala Ala Gly Ser Gly Ala Ala
   65                  70                  75                  80
   Ser Pro Ser Ala Ala Glu Lys Gly Glu Pro Ala Ala Ala Ala Ala Pro
                   85                  90                  95
   Glu Ala Gly Ala Ser Pro Val Glu Lys Glu Ala Pro Ala Glu Gly Glu
               100                 105                 110
   Ala Ala Glu Pro Gly Ser Pro Thr Ala Ala Glu Gly Glu Ala Ala Ser
           115                 120                 125
   Ala Ala Ser Ser Thr Ser Ser Pro Lys Ala Glu Asp Gly Ala Thr Pro
       130                 135                 140
   Ser Pro Ser Asn Glu Thr Pro Lys Lys Lys Lys Lys Arg Phe Ser Phe
   145                 150                 155                 160
   Lys Lys Ser Phe Lys Leu Ser Gly Phe Ser Phe Lys Lys Asn Lys Lys
                   165                 170                 175
   Glu Ala Gly Glu Gly Gly Glu Ala Glu Ala Pro Ala Ala Glu Gly Gly
               180                 185                 190
   Lys Asp Glu Ala Ala Gly Gly Ala Ala Ala Ala Ala Ala Glu Ala Gly
           195                 200                 205
   Ala Ala Ser Gly Glu Gln Ala Ala Ala Pro Gly Glu Glu Ala Ala Ala
       210                 215                 220
   Gly Glu Glu Gly Ala Ala Gly Gly Asp Ser Gln Glu Ala Lys Pro Gln
   225                 230                 235                 240
   Glu Ala Ala Val Ala Pro Glu Lys Pro Pro Ala Ser Asp Glu Thr Lys
                   245                 250                 255
   Ala Ala Glu Glu Pro Ser Lys Val Glu Glu Lys Lys Ala Glu Glu Ala
               260                 265                 270
   Gly Ala Ser Ala Ala Ala Cys Glu Ala Pro Ser Ala Ala Gly Leu Val
           275                 280                 285
   Cys Pro Arg Arg Gly Gly Ser Pro Arg Gly Gly Ala Arg Gly Arg Arg
       290                 295                 300
   Ser Leu Asn Gln Ala Cys Ala Ala Pro Ser Gln Glu Ala Gln Pro Glu
   305                 310                 315                 320
   Cys Ser Pro Glu Ala Pro Pro Ala Glu Ala Ala Glu
                   325                 330
<210> 5
   <211> 2589
   <212> DNA
   <213> Homo sapiens
<220>
   <221> CDS
   <222> (370)..(1368)
<400> 5
   caaccaggga gatttctcca ttttcctctt gtctacagtg cggctacaaa tctgggattt 60
   ttttattact tctttttttt tcgaactaca cttgggctcc tttttttgtg ctcgactttt 120
   ccaccctttt tccctccctc ctgtgctgct gctttttgat ctcttcgact aaaatttttt 180
   tatccggagt gtatttaatc ggttctgttc tgtcctctcc accaccccca cccccctccc 240
   tccggtgtgt gtgccgctgc cgctgttgcc gccgccgctg ctgctgctgc tcgccccgtc 300
   gttacaccaa cccgaggctc tttgtttccc ctcttggatc tgttgagttt ctttgttgaa
   360
   gaagccagc atg ggt gcc cag ttc tcc aag acc gca gcg aag gga gaa gcc
   411
             Met Gly Ala Gln Phe Ser Lys Thr Ala Ala Lys Gly Glu Ala
               1               5                  10
   gcc gcg gag agg cct ggg gag gcg gct gtg gcc tcg tcg cct tcc aaa 459
   Ala Ala Glu Arg Pro Gly Glu Ala Ala Val Ala Ser Ser Pro Ser Lys
   15 20 25 30
   gcg aac gga cag gag aat ggc cac gtg aag gta aac ggc gac gct tcg 507
   Ala Asn Gly Gln Glu Asn Gly His Val Lys Val Asn Gly Asp Ala Ser
   35 40 45
   ccc gcg gcc gcc gag tcg ggc gcc aag gag gag ctg cag gcc aac ggc 555
   Pro Ala Ala Ala Glu Ser Gly Ala Lys Glu Glu Leu Gln Ala Asn Gly
   50 55 60
   agc gcc ccg gcc gcc gac aag gag gag ccc gcg gcc gcc ggg agc ggg 603
   Ser Ala Pro Ala Ala Asp Lys Glu Glu Pro Ala Ala Ala Gly Ser Gly
   65 70 75
   gcg gcg tcg ccc tcc tcg gcc gag aaa ggt gag ccg gcc gcc gcc gct 651
   Ala Ala Ser Pro Ser Ser Ala Glu Lys Gly Glu Pro Ala Ala Ala Ala
   80 85 90
   gcc ccc gag gcc ggg gcc agc ccg gta gag aag gag gcc ccc gcg gaa 699
   Ala Pro Glu Ala Gly Ala Ser Pro Val Glu Lys Glu Ala Pro Ala Glu
   95 100 105 110
   ggc gag gct gcc gag ccc ggc tcg gcc acg gcc gcg gag gga gag gcc 747
   Gly Glu Ala Ala Glu Pro Gly Ser Ala Thr Ala Ala Glu Gly Glu Ala
   115 120 125
   gcg tcg gcc gcc tcc tcg act tct tcg ccc aag gcc gag gac ggg gcc 795
   Ala Ser Ala Ala Ser Ser Thr Ser Ser Pro Lys Ala Glu Asp Gly Ala
   130 135 140
   acg ccc tcg ccc agc aac gag acc ccg aaa aaa aaa aag aag cgc ttt 843
   Thr Pro Ser Pro Ser Asn Glu Thr Pro Lys Lys Lys Lys Lys Arg Phe
   145 150 155
   tcc ttc aag aag tct ttc aag ctg agc ggc ttc tcc ttc aag aag aac 891
   Ser Phe Lys Lys Ser Phe Lys Leu Ser Gly Phe Ser Phe Lys Lys Asn
   160 165 170
   aag aag gag gct gga gaa ggc ggt gag gct gag gcg ccc gct gcc gaa 939
   Lys Lys Glu Ala Gly Glu Gly Gly Glu Ala Glu Ala Pro Ala Ala Glu
   175 180 185 190
   ggc ggc aag gac gag gcc gcc ggg ggc gca gct gcg gcc gcc gcc gag 987
   Gly Gly Lys Asp Glu Ala Ala Gly Gly Ala Ala Ala Ala Ala Ala Glu
   195 200 205
   gcg ggc gcg gcc tcc ggg gag cag gca gcg gcg ccg ggc gag gag gcg 1035
   Ala Gly Ala Ala Ser Gly Glu Gln Ala Ala Ala Pro Gly Glu Glu Ala
   210 215 220
   gca gcg ggc gag gag ggg gcg gcg ggt ggc gac ccg cag gag gcc aag 1083
   Ala Ala Gly Glu Glu Gly Ala Ala Gly Gly Asp Pro Gln Glu Ala Lys
   225 230 235
   ccc cag gag gcc gct gtc gcg cca gag aag ccg ccc gcc agc gac gag 1131
   Pro Gln Glu Ala Ala Val Ala Pro Glu Lys Pro Pro Ala Ser Asp Glu
   240 245 250
   acc aag gcc gcc gag gag ccc agc aag gtg gag gag aaa aag gcc gag
   1179
   Thr Lys Ala Ala Glu Glu Pro Ser Lys Val Glu Glu Lys Lys Ala Glu
   255                 260                 265                 270
   gag gcc ggg gcc agc gcc gcc gcc tgc gag gcc ccc tcc gcc gcc ggg
   1227
   Glu Ala Gly Ala Ser Ala Ala Ala Cys Glu Ala Pro Ser Ala Ala Gly
                   275                 280                 285
   ccc ggc gcg ccc ccg gag cag gag gca gcc ccc gcg gag gag ccc gcg
   1275
   Pro Gly Ala Pro Pro Glu Gln Glu Ala Ala Pro Ala Glu Glu Pro Ala
               290                 295                 300
   gcc gcc gca gcc tcg tca gcc tgc gca gcc ccc tca cag gag gcc cag
   1323
   Ala Ala Ala Ala Ser Ser Ala Cys Ala Ala Pro Ser Gln Glu Ala Gln
           305                 310                 315
   ccc gag tgc agt cca gaa gcc ccc cca gcg gag gcg gca gag taa
   1368
   Pro Glu Cys Ser Pro Glu Ala Pro Pro Ala Glu Ala Ala Glu
       320                 325                 330
   aagagcaagc ttttgtgaga taatcgaaga acttttctcc cccgtttgtt tgttggagtg
   1428
   gtgccaggta ctgttttgga gaacttgtct acaaccaggg attgatttta aagatgtctt
   1488
   tttttatttt actttttttt aagcaccaaa ttttgttgtt tttttttttc tcccctcccc
   1548
   acagatccca tctcaaatca ttctgttaac caccattcca acaggtcgag gagagcttaa
   1608
   acaccttctt cctctgcctt gtttctcttt tattttttat tttttcgcat cagtattaat
   1668
   gtttttgcat actttgcatc tttattcaaa agtgtaaact ttctttgtca atctatggac
   1728
   atgcccatat atgaaggaga tgggtgggtc aaaaagggat atcaaatgaa gtgatagggg
   1788
   tcacaatggg gaaattgaag tggtgcataa cattgccaaa atagtgtgcc actagaaatg
   1848
   gtgtaaaggc tgtctttttt ttttttttta aagaaaagtt attaccatgt attttgtgag
   1908
   gcaggtttac aacactacaa gtcttgagtt aagaaggaaa gaggaaaaaa gaaaaaacac
   1968
   caatacccag atttaaaaaa aaaaaaacga tcatagtctt aggagttcat ttaaaccata
   2028
   ggaacttttc acttatctca tgttagctgt accagtcagt gattaagtag aactacaagt
   2088
   tgtataggct ttattgttta ttgctggttt atgaccttaa taaagtgtaa ttatgtatta 2148
   ccagcagggt gtttttaact gtgactattg tataaaaaca aatcttgata tccagaagca 2208
   catgaagttt gcaactttcc accctgccca tttttgtaaa actgcagtca tcttggacct 2268
   tttaaaacac aaattttaaa ctcaaccaag ctgtgataag tggaatggtt actgtttata 2328
   ctgtggtatg tttttgatta cagcagataa tgctttcttt tccagtcgtc tttgagaata 2388
   aaggaaaaaa aatcttcaga tgcaatggtt ttgtgtagca tcttgtctat catgttttgt 2448
   aaatactgga gaagctttga ccaatttgac ttagagatgg aatgtaactt tgcttacaaa 2508
   aattgctatt aaactcctgc ttaaggtgtt ctaattttct gtgagcacac taaaagcgaa 2568
   aaataaatgt gaataaaatg t 2589
<210> 6
   <211> 332
   <212> PRT
   <213> Homo sapiens
<400> 6
   Met Gly Ala Gln Phe Ser Lys Thr Ala Ala Lys Gly Glu Ala Ala Ala
   1               5                   10                  15
   Glu Arg Pro Gly Glu Ala Ala Val Ala Ser Ser Pro Ser Lys Ala Asn
               20                  25                  30
   Gly Gln Glu Asn Gly His Val Lys Val Asn Gly Asp Ala Ser Pro Ala
           35                  40                  45
   Ala Ala Glu Ser Gly Ala Lys Glu Glu Leu Gln Ala Asn Gly Ser Ala
       50                  55                  60
   Pro Ala Ala Asp Lys Glu Glu Pro Ala Ala Ala Gly Ser Gly Ala Ala
   65                  70                  75                  80
   Ser Pro Ser Ser Ala Glu Lys Gly Glu Pro Ala Ala Ala Ala Ala Pro
                   85                  90                  95
   Glu Ala Gly Ala Ser Pro Val Glu Lys Glu Ala Pro Ala Glu Gly Glu
               100                 105                 110
   Ala Ala Glu Pro Gly Ser Ala Thr Ala Ala Glu Gly Glu Ala Ala Ser
           115                 120                 125
   Ala Ala Ser Ser Thr Ser Ser Pro Lys Ala Glu Asp Gly Ala Thr Pro
       130                 135                 140
   Ser Pro Ser Asn Glu Thr Pro Lys Lys Lys Lys Lys Arg Phe Ser Phe
   145                 150                 155                 160
   Lys Lys Ser Phe Lys Leu Ser Gly Phe Ser Phe Lys Lys Asn Lys Lys
                   165                 170                 175
   Glu Ala Gly Glu Gly Gly Glu Ala Glu Ala Pro Ala Ala Glu Gly Gly
               180                 185                 190
   Lys Asp Glu Ala Ala Gly Gly Ala Ala Ala Ala Ala Ala Glu Ala Gly
           195                 200                 205
   Ala Ala Ser Gly Glu Gln Ala Ala Ala Pro Gly Glu Glu Ala Ala Ala
       210                 215                 220
   Gly Glu Glu Gly Ala Ala Gly Gly Asp Pro Gln Glu Ala Lys Pro Gln
   225                 230                 235                 240
   Glu Ala Ala Val Ala Pro Glu Lys Pro Pro Ala Ser Asp Glu Thr Lys
                   245                 250                 255
   Ala Ala Glu Glu Pro Ser Lys Val Glu Glu Lys Lys Ala Glu Glu Ala
               260                 265                 270
   Gly Ala Ser Ala Ala Ala Cys Glu Ala Pro Ser Ala Ala Gly Pro Gly
           275                 280                 285
   Ala Pro Pro Glu Gln Glu Ala Ala Pro Ala Glu Glu Pro Ala Ala Ala
       290                 295                 300
   Ala Ala Ser Ser Ala Cys Ala Ala Pro Ser Gln Glu Ala Gln Pro Glu
   305                 310                 315                 320
   Cys Ser Pro Glu Ala Pro Pro Ala Glu Ala Ala Glu
                   325                 330

## Claims

1. A peptide consisting of from 5 to fewer than 50 contiguous amino acids from an N-terminal sequence of SEQ ID NO: 4 wherein said peptide inhibits MARCKS protein-related mucus secretion and wherein said peptide reduces mucus secretion by a mucus-secreting cell as compared to that which would occur in the absence of said peptide.

2. A peptide consisting of from 10 to 50 contiguous amino acids from an N-terminal sequence of SEQ ID NO: 4 wherein said peptide inhibits MARCKS protein-related mucus secretion and wherein said peptide reduces mucus secretion by a mucus-secreting cell as compared to that which would occur in the absence of said peptide.

3. A peptide according to any of the preceding claims wherein said peptide blocks the secretion of inflammatory mediators from cells and decreases inflammation.

4. The peptide according to any of the preceding claims, wherein the peptide is myristoylated.

5. The peptide of any of claims 1 to 4, wherein the peptide has an amino acid sequence comprising SEQ ID NO: 1.

6. A myristoylated peptide of the N-terminal region of MARCKS protein, wherein the peptide consists of an amino acid sequence that is identical to a contiguous sequence of 10 to 50 amino acids from an N-terminal sequence of SEQ ID NO: 4, wherein said peptide reduces mucus secretion by a mucus-secreting cell as compared to that which would occur in the absence of said peptide.

7. A myristoylated peptide consisting of sequence;
Myristic acid-GAQFSKTAAKGEAAAERPGEAAVA (SEQ ID NO: 1) or myristoylated fragments thereof consisting of at least 10 amino acids wherein said peptide inhibits MARCKS protein-related mucus secretion and wherein said peptide reduces mucus secretion by a mucus-secreting cell as compared to that which would occur in the absence of said peptide.

8. A myristoylated peptide consisting of sequence;
Myristic acid-GAQFSKTAAKGEAA.AERPGEAAVA (SEQ ID NO:1) or myristoylated fragments thereof consisting of at least 5 amino acids wherein said peptide inhibits MARCKS protein-related mucus secretion and wherein said peptide reduces mucus secretion by a mucus-secreting cell as compared to that which would occur in the absence of said peptide.

9. A peptide according to any of claims 1 to 8 for use as a medicament.

10. A pharmaceutical composition comprising the peptide of any one of claims 4, 6, 7 or claim 8 and a pharmaceutically acceptable carrier.

11. Use of a peptide according to any of claims 1 to 8 for the manufacture of a medicament for the therapeutic or prophylactic treatment of mucus secretion.

12. Use of a peptide according to any of claims 1 to 8 for the manufacture of a medicament for the therapeutic or prophylactic treatment of inflammation.

13. Use of a peptide according to any of claims 1 to 8 for the manufacture of a medicament for the therapeutic or prophylactic treatment of mucus secretion and inflammation.

14. Use of a peptide according to any of claims 1 to 8 for the manufacture of a medicament for the therapeutic or prophylactic treatment of airway disease.

15. The peptide of any of claims 1 to 8 for use in treating mucus secretion.

16. The peptide of any of claims 1 to 8 for use in treating inflammation.

17. The peptide of any of claims 1 to 8 for use in treating mucus secretion and inflammation.

18. The peptide of any of claims 1 to 8 for use in treating airway disease.

## Patentansprüche

1. Peptid bestehend aus 5 bis weniger als 50 zusammenhängenden Aminosäuren einer N-terminalen Sequenz der SEQ ID NR: 4, wobei das Peptid die MARCKS-Protein abhängige Mucus-Sekretion hemmt und wobei das Peptid die Mucus-Sekretion einer Mucus sezernierenden Zelle im Vergleich mit dem, was in Abwesenheit des Proteins stattfinden würde, reduziert.

2. Peptid bestehend aus 10 bis 50 zusammenhängenden Aminosäuren einer N-terminalen Sequenz der SEQ ID NR: 4, wobei das Peptid die MARCKS-Protein abhängige Mucus-Sekretion hemmt und wobei das Peptid die Mucus-Sekretion einer Mucus sezernierenden Zelle im Vergleich mit dem, was in Abwesenheit des Proteins stattfinden würde, reduziert.

3. Peptid nach einem der vorhergehenden Ansprüche, wobei das Peptid die Sekretion von Entzündungsmediatoren aus Zellen hemmt und Entzündungen abmindert.

4. Peptid nach einem der vorhergehenden Ansprüche, wobei das Peptid myristoyliert ist.

5. Peptid nach einem der Ansprüche 1 bis 4, wobei das Peptid eine Aminosäuresequenz aufweist, welche die SEQ ID NR: 1 umfasst.

6. Myristoyliertes Peptid aus der N-terminalen Region des MARCKS-Proteins, wobei das Peptid aus einer Aminosäuresequenz besteht, die mit einer zusammenhängenden Sequenz von 10 bis 50 Aminosäuren einer N-terminalen Sequenz der SEQ ID NR: 4 identisch ist, wobei das Peptid die Mucus-Sekretion einer Mucus sezernierenden Zelle im Vergleich mit dem, was in Abwesenheit des Proteins stattfinden würde, reduziert.

7. Myristoyliertes Peptid bestehend aus der Sequenz
Myristinsäure-GAQFSKTAAKGEAAAERPGEAAVA (SEQ ID NR:1) oder myristoylierte Fragmente davon, die aus mindestens 10 Aminosäuren bestehen, wobei das Peptid die MARCKS-Protein abhängige Mucus-Sekretion hemmt und wobei das Peptid die Mucus-Sekretion einer Mucus sezernierenden Zelle im Vergleich mit dem, was in Abwesenheit des Proteins stattfinden würde, reduziert.

8. Myristoyliertes Peptid bestehend aus der Sequenz
Myristinsäure-GAQFSKTAAKGEAAAERPGEAAVA (SEQ ID NR:1) oder myristoylierte Fragmente davon, die aus mindestens 5 Aminosäuren bestehen, wobei das Peptid die MARCKS-Protein abhängige Mucus-Sekretion hemmt und wobei das Peptid die Mucus-Sekretion einer Mucus sezernierenden Zelle im Vergleich mit dem, was in Abwesenheit des Proteins stattfinden würde, reduziert.

9. Peptid nach einem der Ansprüche 1 bis 8 zur Verwendung als Medikament.

10. Pharmazeutische Zusammensetzung, welche das Peptid nach einem der Ansprüche 4, 6, 7 oder Anspruch 8 und einen pharmazeutisch zulässigen Träger umfasst.

11. Verwendung eines Peptids nach einem der Ansprüche 1 bis 8 zur Herstellung eines Medikaments zur therapeutischen oder prophylaktischen Behandlung der Mucus-Sekretion.

12. Verwendung eines Peptids nach einem der Ansprüche 1 bis 8 zur Herstellung eines Medikaments zur therapeutischen oder prophylaktischen Behandlung von Entzündungen.

13. Verwendung eines Peptids nach einem der Ansprüche 1 bis 8 zur Herstellung eines Medikaments zur therapeutischen oder prophylaktischen Behandlung von Mukus-Sekretion und -Entzündung.

14. Verwendung eines Peptids nach einem der Ansprüche 1 bis 8 zur Herstellung eines Medikaments zur therapeutischen oder prophylaktischen Behandlung von Atemwegserkrankungen.

15. Peptid nach einem der Ansprüche 1 bis 8 zur Verwendung bei der Behandlung der Mucus-Sekretion.

16. Peptid nach einem der Ansprüche 1 bis 8 zur Verwendung bei der Behandlung von Entzündungen.

17. Peptid nach einem der Ansprüche 1 bis 8 zur Verwendung bei der Behandlung von Mukus-Sekretion und -Entzündung.

18. Peptid nach einem der Ansprüche 1 bis 8 zur Verwendung bei der Behandlung von Atemwegserkrankungen.

## Revendications

1. Peptide constitué de 5 à moins de 50 acides aminés contigus provenant d'une séquence N-terminale de SEQ ID NO : 4, où ledit peptide inhibe la sécrétion de mucus associée à la protéine MARCKS et où ledit peptide réduit la sécrétion de mucus par une cellule sécrétrice de mucus comparativement à celle qui se produira en l'absence dudit peptide.

2. Peptide constitué de 10 à 50 acides aminés contigus provenant d'une séquence N-terminale de SEQ ID NO : 4, où ledit peptide inhibe la sécrétion de mucus associée à la protéine MARCKS et où ledit peptide réduit la sécrétion de mucus par une cellule sécrétrice de mucus comparativement à celle qui se produira en l'absence dudit peptide.

3. Peptide selon l'une quelconque des revendications précédentes, où ledit peptide bloque la sécrétion de médiateurs inflammatoires à partir des cellules et diminue l'inflammation.

4. Peptide selon l'une quelconque des revendications précédentes, où le peptide est myristoylé.

5. Peptide selon l'une quelconque des revendications 1 à 4, où le peptide a une séquence d'acides aminés comprenant SEQ ID NO : 1.

6. Peptide myristoylé de la région N-terminale de la protéine MARCKS, où le peptide est constitué d'une séquence d'acides aminés qui est identique à une séquence contiguë de 10 à 50 acides aminés provenant d'une séquence N-terminale de SEQ ID NO : 4, où ledit peptide réduit la sécrétion de mucus par une cellule sécrétrice de mucus comparativement à celle qui se produira en l'absence dudit peptide.

7. Peptide myristoylé constitué de la séquence :
acide myristique-GAQFSKTAAKGEAAAERPGEAAVA (SEQ ID NO : 1) ou de fragments myristoylés de celle-ci constitués d'au moins 10 acides aminés, où ledit peptide inhibe la sécrétion de mucus associée à la protéine MARCKS et où ledit peptide réduit la sécrétion de mucus par une cellule sécrétrice de mucus comparativement à celle qui apparaîtra en l'absence dudit peptide.

8. Peptide myristoylé constitué de la séquence :
acide myristique-GAQFSKTAAKGEAAAERPGEAAVA (SEQ ID NO : 1) ou de fragments myristoylés de celle-ci constitués d'au moins 5 acides aminés, où ledit peptide inhibe la sécrétion de mucus associée à la protéine MARCKS et où ledit peptide réduit la sécrétion de mucus par une cellule sécrétrice de mucus comparativement à celle qui apparaîtra en l'absence dudit peptide.

9. Peptide selon l'une quelconque des revendications 1 à 8, pour une utilisation en tant que médicament.

10. Composition pharmaceutique comprenant le peptide selon l'une quelconque des revendications 4, 6, 7 ou la revendication 8 et un support pharmaceutiquement acceptable.

11. Utilisation d'un peptide selon l'une quelconque des revendications 1 à 8, pour la fabrication d'un médicament destiné au traitement thérapeutique ou prophylactique de la sécrétion de mucus.

12. Utilisation d'un peptide selon l'une quelconque des revendications 1 à 8, pour la fabrication d'un médicament destiné au traitement thérapeutique ou prophylactique de l'inflammation.

13. Utilisation d'un peptide selon l'une quelconque des revendications 1 à 8, pour la fabrication d'un médicament destiné au traitement thérapeutique ou prophylactique de la sécrétion de mucus et de l'inflammation.

14. Utilisation d'un peptide selon l'une quelconque des revendications 1 à 8, pour la fabrication d'un médicament destiné au traitement thérapeutique et/ou prophylactique des maladies des voies respiratoires.

15. Peptide selon l'une quelconque des revendications 1 à 8, pour une utilisation dans le traitement de la sécrétion de mucus.

16. Peptide selon l'une quelconque des revendications 1 à 8, pour une utilisation dans le traitement de l'inflammation.

17. Peptide selon l'une quelconque des revendications 1 à 8, pour une utilisation dans le traitement de la sécrétion de mucus et de l'inflammation.

18. Peptide selon l'une quelconque des revendications 1 à 8, pour une utilisation dans le traitement des maladies des voies respiratoires.
